# FASCICULE DE BREVET EUROPEEN

(11) **EP 0 876 482 B1**
(45) Date de publication et mention de la délivrance du brevet: **19.03.2003**
(21) Numéro de dépôt: 96938273.8
(22) Date de dépôt: 07.11.1996
(51) Int. Cl.: C12N 15/12, C07K 14/715, C12N 15/85, C12N 5/10, C12Q 1/68, C12N 15/11, G01N 33/53, C07K 16/28, G01N 33/577, G01N 33/68, A61K 38/17

(54) **POLYPEPTIDE RECEPTEUR DE L'IL-I3**
IL-13 REZEPTOR POLYPEPTID
IL-13 RECEPTOR POLYPEPTIDE

(30) Priorité: 06.12.1995 FR 9514424
(43) Date de publication de la demande: 11.11.1998
(73) Titulaire: SANOFI-SYNTHELABO, 75635 Paris Cédex 13 (FR)
(72) Inventeur: CAPUT, Daniel, F-31290 Avignolet-Lauragais (FR); FERRARA, Pascual, F-31290 Avignolet-Lauragais (FR); LAURENT, Patrick, F-31190 Auterive (FR); VITA, Natalio, F-31450 Montgiscard (FR)
(74) Mandataire: Bernasconi, Jean Raymond
(86) Numéro de dépôt international: FR9601756
(87) Numéro de publication internationale: WO97020926

(56) Documents cités:
- FEBS LETTERS, vol. 366, no. 2-3, 12 Juin 1995, AMSTERDAM, PAYS-BAS, pages 122-126, XP002010891 S. LEFORT ET AL.: "IL-13 and IL-4 share signal transduction elements as well as receptor components in TF-1 cells." cité dans la demande
- THE JOURNAL OF BIOLOGICAL CHEMISTRY, vol. 270, no. 8, 24 Février 1995, BALTIMORE, MD, TATS-UNIS, pages 3512-3517, XP002010892 N. VITA ET AL.: "Characterization and comparison of the interleukin 13 receptor with the interleukin 4 receptor on several cell types." cité dans la demande
- THE JOURNAL OF BIOLOGICAL CHEMISTRY, vol. 270, no. 15, 14 Avril 1995, BALTIMORE, MD, TATS-UNIS, pages 8797-8804, XP002010893 N. OBIRI ET AL.: "Receptor for interleukin 13." cité dans la demande
- THE JOURNAL OF BIOLOGICAL CHEMISTRY, vol. 270, no. 23, 9 Juin 1995, BALTIMORE, MD, TATS-UNIS, pages 13869-13878, XP002010894 S. ZURAWSKI ET AL.: "The primary binding subunit of the human interleukin-4 receptor is also a component of the interleukin-13 receptor."
- THE JOURNAL OF BIOLOGICAL CHEMISTRY, vol. 271, no. 46, 15 Novembre 1996, BALTIMORE, MD, TATS-UNIS, pages 29265-29270, XP002025818 M. JAVAD AMAN ET AL.: "cDNA cloning and characterization of the human interleukin 13 receptor alpha chain."
- THE JOURNAL OF BIOLOGICAL CHEMISTRY, vol. 271, no. 28, 12 Juillet 1996, BALTIMORE, MD, TATS-UNIS, pages 16921-16926, XP002025819 D. CAPUT ET AL.: "Cloning and characterization of a specific interleukin (IL)-13 binding protein structurally related to the IL-5 receptor alpha chain."
- IMMUNOLOGY TODAY, vol. 17, no. 3, Mars 1996, AMSTERDAM, PAYS-BAS, pages 108-110, XP002010895 R. CALLARD ET AL.: "IL-4 and IL-13 receptors: are they one and the same?"

## Description

La présente invention concerne des polypeptides purifiés ayant une activité de récepteur spécifique de l'interleukine 13 (IL-13), leurs fragments biologiquement actifs et les séquences d'acides nucléiques correspondantes, et leurs applications.

L'IL-13 est une cytokine de 112 acides aminés récemment identifiée (1,2), sécrétée par des lymphocytes T activés, les lymphocytes B et les mastocytes après activation.

De par ses nombreuses propriétés biologiques communes avec l'IL-4, l'IL-13 a été décrite comme étant une cytokine "IL-4-like". Ses activités sont en effet similaires à celles de l'IL-4 sur les cellules B (3-5), les monocytes (6-10) et autres cellules non hématopoïétiques (11-12). En revanche, contrairement à l'IL-4, elle n'exercerait pas d'effet particulier sur des cellules T au repos ou activées (13).

Les différentes activités biologiques de l'IL-13 sur les monocytes/macrophages, les lymphocytes B et certains précurseurs hématopoïétiques ont été décrites en détail par A.J. Minty, ainsi que dans des articles de revues sur l'IL-13 (voir par ex. 14). Plusieurs données indiquent en outre que cette cytokine a un effet pleïotrope sur d'autres types cellulaires. Ces cellules non-hématopoïétiques affectées directement par l'IL-13 sont des cellules endothéliales et microgliales, des kératinocytes, et des carcinomes de rein et de colon.

Les activités anti-inflammatoires et immunorégulatrices de l'IL-13 peuvent être utiles par exemple dans le traitement des pathologies auto-immunes, tumorales et virales.

Une mise à profit de ces propriétés biologiques au plan clinique nécessite toutefois une parfaite connaissance des signaux et mécanismes par lesquels ces effets sont exercés, de façon à pouvoir les contrôler et les moduler dans les pathologies concernées.

L'une des étapes dans l'analyse du signal transmis par une molécule biologique au sein d'une cellule, consiste à identifier son récepteur membranaire. Les travaux réalisés à cet effet sur le récepteur de l'IL-13 ont montré que l'IL-13 et l'IL-4 avaient en commun un récepteur, ou tout au moins certains des composants d'un complexe récepteur, ainsi que des éléments de transduction de signaux (15-18). Ce récepteur est présent à la surface de différents types cellulaires, en nombre variable selon le type cellulaire considéré. La distribution comparative des récepteurs de l'IL-13 et de l'IL-4 a été indiquée par A.J. Minty (14).

Kondo et al. (19) ont décrit la structure d'un récepteur de haute affinité pour l'IL-4. Celui-ci est un dimère, formé par l'association d'une glycoprotéine de 140 kDa (IL-4R) et de la chaîne γ du récepteur de l'IL-2 (γc). L'IL-4 peut se lier à la sous-unité glycoprotéique de 140 kDa (IL-4R ou gp 140) avec une bonne affinité (Kd comprise entre 50 et 100 pM) (15). Cependant, cette affinité est augmentée d'un facteur 2 à 3, lorsque la chaîne γc est associée à la gp 140. Cette association est en outre nécessaire à la transmission de certains signaux médiés par l'IL-4 (19,20).

Des expériences de compétition croisée pour la liaison, soit de l'IL-13, soit de l'IL-4, ont démontré que l'IL-4 peut normalement empêcher la liaison de l'IL-13, alors que l'IL-13 ne peut généralement empêcher que partiellement la liaison de l'IL-4 à son récepteur (17,21), et ne se fixe sur aucune des deux sous-unités du récepteur de l'IL-4, ni sur le complexe formé par leur association. Sur la base de ces observations, les auteurs de la présente invention ont supposé que le récepteur spécifique de l'IL-13 était constitué du complexe récepteur IL-4 associé avec un autre composant liant l'IL-13, (IL-13Rβ).

Des travaux réalisés sur une lignée cellulaire érythroleucémique capable de proliférer en réponse à l'IL-13 et à l'IL-4 (lignée TF-1), leur ont permis de montrer que ces deux cytokines produisaient des événements intracellulaires similaires après fixation sur leur récepteur (18). En parallèle, des expériences de pontage ("cross-link") leur ont permis de montrer que la gp 140 pouvait former des hétérodimères soit avec la chaîne γ, soit avec une nouvelle sous-unité, d'un poids moléculaire de 55 à 70 kDa (17,21).

D'autre part des travaux réalisés récemment sur une lignée de cellules souches embryonnaires de souris ont permis d'isoler l'ADN génomique et l'ADNc encodant pour un polypeptide de 424 résidus d'acides aminés (IL-13Rα), suggérant que le récepteur de l'IL-13 partageait avec le récepteur de l'IL-4 une chaîne commune pour constituer un récepteur à haute affinité (22,23), c'est-à-dire présente une affinité dont la constante Kd est située entre des valeurs comprises entre 10 pM et 100 pM environ (un récepteur de basse affinité présentant une constante Kd située entre des valeurs comprises entre 2nM et 10 nM).

Compte tenu de l'importance au plan médical de la compréhension fine des phénomènes de régulation de l'IL-4 et de l'IL-13, et en particulier de la possibilité de pouvoir séparer et contrôler isolément les effets produits par l'une ou l'autre de ces deux cytokines, les auteurs de la présente invention se sont intéressés, d'une part, à la caractérisation d'un polypeptide liant spécifiquement l'IL-13 avec une haute affinité et, d'autre part, à la caractérisation d'un autre polypeptide qui, seul, lie spécifiquement l'IL-13 avec une basse affinité et qui, s'il est associé avec le récepteur de l'IL-4, constitue un récepteur de haute affinité pour l'IL-13.

Ceux-ci ont à présent identifié une lignée cellulaire de carcinome humain exprimant le récepteur spécifique de l'IL-13 en quantité plus importante que d'autres lignées de carcinome rénal humaines connues (21), et ont réalisé le clonage de la sous-unité primaire responsable de la fixation de l'IL-13 sur le récepteur de l'IL-4/IL-13, dénommée IL-13 Rβ, ainsi que le clonage de la chaine commune que partage le récepteur de l'IL-13 et le récepteur de l'IL-4 pour constituer un récepteur de haute affinité permettant une compétition croisée des 2 cytokines, dénommée IL-13Rα.

La présente invention concerne donc des polypeptides purifiés liant spécifiquement l'IL-13.

Plus particulièrement, l'invention a pour objet des polypeptides purifiés dont les séquences d'acides aminés correspondent à celle d'un récepteur spécifique de l'IL-13 (IL-13Rβ et IL-13Rα), ou des fragments biologiquement actifs de ceux-ci.

L'invention a également pour objet des séquences d'ADN isolées codant pour lesdits polypeptides ou leurs fragments biologiquement actifs.

Elle vise en outre les vecteurs d'expression contenant au moins l'une des séquences nucléotidiques définies ci-dessus, et les cellules hôtes transfectées par ces vecteurs d'expression dans des conditions permettant la réplication et/ou l'expression de l'une desdites séquences nucléotidiques.

Les méthodes de production de l'IL-13Rβ et de l'IL-13Rα recombinants ou de leurs fragments biologiquement actifs par les cellules hôtes transfectées font également partie de l'invention.

L'invention comprend également des compositions pharmaceutiques comprenant l'IL-13Rβ ou/et l'IL-13Rα ou des fragments biologiquement actifs de ceux-ci pour la régulation des mécanismes immunologiques et inflammatoires produits par l'IL-13. Elle vise en outre une méthode pour l'identification d'agents capables de moduler l'activité de l'IL-13Rβ ou/et de l'IL-13Rα, et l'utilisation de I'IL-R13β ou/et de l'IL-13Rα ou de fragments de ceux-ci pour le criblage de ces agents ainsi que pour la fabrication de nouveaux produits capables de moduler l'activité du récepteur de l'IL-13.

L'invention comprend également des anticorps ou des dérivés d'anticorps spécifiques de l'IL-13Rβ ou/et de l'IL-13Rα.

Elle vise enfin une méthode de traitement thérapeutique pour moduler les réactions immunologiques médiées par l'IL-13, comprenant l'administration à un patient de l'IL-13Rβ ou/et de l'IL-13Rα ou d'un de leurs fragments biologiquement actif ou d'un composé capable de moduler spécifiquement l'activité de ce récepteur, en association avec un véhicule pharmaceutiquement acceptable.

Dans la description de l'invention ci-après, on utilise les définitions suivantes :
- polypeptide liant spécifiquement l'IL-13 avec une haute affinité (IL-13Rβ) : un polypeptide comprenant la séquence d'acides aminés SEQ ID n° 2 ou tout fragment ou dérivé de celui-ci biologiquement actif;
- polypeptide qui, seul, lie spécifiquement l'IL-13 avec une basse affinité et qui, s'il est associé avec le récepteur de l'IL-4, constitue un récepteur de haute affinité (IL-13Rα) : un polypeptide comprenant la séquence d'acides aminés SEQ ID N° 4 ou tout fragment ou dérivé de celui-ci biologiquement actif;
- biologiquement actif : capable de se lier spécifiquement à l'IL-13 et/ou de participer à la transduction du signal spécifiquement produit par l'IL-13 au niveau de la membrane cellulaire, et/ou capable d'interagir avec le récepteur spécifique de l'IL-4 (IL-4R/gp 140) pour former un complexe capable de lier l'IL-4 et l'IL-13, et/ou qui est reconnu par des anticorps spécifiques du polypeptide de séquence SEQ ID n° 2 ou/et de séquence SEQ ID n°4, et/ou capable d'induire des anticorps qui reconnaissent le polypeptide de séquence SEQ ID n° 2 ou/et de séquence SEQ ID n°4;
- dérivé : tout polypeptide variant du polypeptide de séquence SEQ ID n° 2 ou/et de séquence SEQ ID n°4, ou toute molécule résultant d'une modification de nature génétique et/ou chimique de la séquence SEQ ID n° 2 ou de séquence SEQ ID n°4, c'est-à-dire obtenue par mutation, délétion, addition, substitution et/ou modification chimique d'un seul ou d'un nombre limité d'acides aminés, ainsi que toute séquence isoforme, c'est-à-dire une séquence identique à la séquence SEQ ID n° 2 ou à la séquence SEQ ID n°4, à l'un de leurs fragments ou à l'une de leurs séquences modifiées, contenant un ou plusieurs acides aminés sous la forme d'énantiomère D, lesdites séquences variantes, modifiées ou isoformes ayant conservé au moins l'une des propriétés les rendant biologiquement actives.

La présente invention a pour objet un polypeptide purifié, comprenant une séquence d'acides aminés choisie parmi :
a) la séquence SEQ ID n° 2 ou la séquence SEQ ID n°4,
b) toute séquence biologiquement active dérivée de SEQ ID n° 2 ou de SEQ ID n°4, selon la définition donnée précédemment.

La fabrication de dérivés peut avoir différents objectifs, dont en particulier celui d'augmenter l'affinité du récepteur pour l'IL-13, celui de moduler la compétition croisée entre l'IL-13 et l'IL-4, celui d'améliorer leurs taux de production, d'augmenter leur résistance à des protéases, de modifier leur activité biologique ou de leur conférer de nouvelles propriétés pharmaceutiques et/ou biologiques.

Parmi des variants biologiquement actifs des polypeptides tels que définis précédemment, on préfère les fragments produits par épissage alternatif des transcripts (ARN messagers) du gène codant pour l'une des séquences d'acides aminés décrites ci-dessus.

Dans une variante avantageuse, les 8 acides aminés C-terminaux du polypeptide de séquence SEQ ID n° 2 sont substitués par les 6 acides aminés suivants : VRCVTL.

Selon un autre aspect avantageux, l'invention vise une forme soluble de l'IL-13Rβ, dénommée IL-13Rβs, comprenant notamment le domaine extra-cellulaire du polypeptide de séquence SEQ ID n° 2 s'étendant jusqu'au résidu 343 et préférentiellement jusqu'au résidu 337 ainsi qu'une forme soluble de l'IL-13Rα, dénommée IL-13Rαs, comprenant notamment le domaine extracellulaire du polypeptide de séquence ID n°4 s'étendant jusqu'au résidu 343 et préférentiellement jusqu'aux résidus compris entre 336 et 342.

Le polypeptide qui comprend la séquence SEQ ID n° 2 ou la séquence SEQ ID n°4 représente un mode de réalisation particulier de l'invention. Comme cela apparaîtra dans les exemples, ce polypeptide peut être exprimé à la surface de cellules humaines pour former un récepteur de l'IL-13 fonctionnel ou s'associer avec le récepteur de l'IL-4 pour former, avec la chaîne γ du récepteur de l'IL-2 le complexe récepteur commun à l'IL-4 et l'IL-13.

La présente invention a également pour objet une séquence d'acides nucléiques isolée, choisie parmi:
a) la séquence SEQ ID n° 1,
b) la séquence SEQ ID n°3,
c) les séquences d'acides nucléiques capables de s'hybrider à la séquence SEQ ID n° 1 ou à la séquence SEQ ID n°3, ou à leurs séquences complémentaires et codant pour des polypeptides ayant une activité de récepteur de l'IL-13, ou permettant de reconstituer un récepteur de haute affinité pour l'IL-13 et l'IL-4,
d) les séquences d'acides nucléiques dérivées des séquences a) et b) et c) du fait de la dégénérescence du code génétique.

Plus particulièrement, l'invention a pour objet une séquence codant pour la partie soluble de l'IL-13Rβ ou de l'IL-13Rα et tout variant produit par épissage alternatif des transcripts de l'IL-13Rβ ou de l'IL-13Rα, conservant au moins une des propriétés biologiques décrites.

Un mode de réalisation préféré est représenté par une séquence d'acides nucléiques comprenant ou constituée par l'enchaînement de nucléotides s'étendant du nucléotide n° 1 jusqu'au nucléotide 1081, et préférentiellement jusqu'au nucléotide 1063 sur la séquence SEQ ID n° 1.

Un autre mode de réalisation préféré est représenté par une séquence d'acides nucléiques comprenant ou constitué par l'enchainement de nucléotides s'étendant du nucléotide n°1 jusqu'au nucléotide n° 1059, et préférentiellement jusqu'aux nucléotides compris entre les numéros 1041 et 1056 sur la séquence SEQ ID n° 3.

Avantageusement, la séquence d'acides nucléiques selon l'invention est une séquence codant pour une protéine correspondant à la forme mature de l'IL-13Rβ ou de l'IL-13Rα, cette protéine mature étant le résultat de la libération du peptide signal.

Les différentes séquences nucléotidiques de l'invention peuvent être d'origine artificielle ou non. Il peut s'agir de séquences d'ADN ou d'ARN, obtenues par criblage de banques de séquences au moyen de sondes élaborées sur la base de la séquence SEQ ID n° 1 ou de la séquence SEQ ID n°3. De telles banques peuvent être préparées par des techniques classiques de biologie moléculaire, connues de l'homme de l'art.

Les séquences nucléotidiques selon l'invention peuvent également être préparées par synthèse chimique, ou encore par des méthodes mixtes incluant la modification chimique ou enzymatique de séquences obtenues par criblage des banques.

Ces séquences nucléotidiques permettent la réalisation de sondes nucléotidiques, codant pour un polypeptide selon l'invention ou un fragment biologiquement actif de celui-ci. Les conditions d'hybridation appropriées correspondent aux conditions de température et de force ionique usuellement utilisées par l'homme du métier, de préférence à des conditions de température comprises entre (Tₘ moins 5°C) et (Tₘ moins 30°C) et de préférence encore, à des conditions de température comprises entre (Tₘ moins 5°C) et (Tₘ moins 10°C) (forte stringence), Tₘ étant la température de fusion, définie comme étant la température à laquelle 50 % des brins appariés se séparent. De telles sondes font également partie de l'invention. Elles peuvent être utilisées comme outil de diagnostic *IN VITRO* pour la détection, par des expériences d'hybridation, de transcripts spécifiques des polypeptides de l'invention dans des échantillons biologiques ou pour la mise en évidence de synthèses aberrantes ou d'anomalies génétiques résultant d'un polymorphisme, de mutations ou d'un mauvais épissage.

Les sondes de l'invention comportent au minimum 10 nucléotides, et au maximum comportent la totalité de la séquence nucléotidique SEQ ID n° 1 ou de SEQ ID n°3 ou de leur brin complémentaire.

Parmi les sondes les plus courtes, c'est-à-dire d'environ 10 à 15 nucléotides, les conditions d'hybridation appropriées correspondent aux conditions de température et de force ionique usuellement utilisées par l'homme du métier.

Préférentiellement, les sondes de l'invention sont marquées, préalablement à leur utilisation. Pour cela, plusieurs techniques sont à la portée de l'homme du métier comme par exemple le marquage fluorescent, radioactif, chimioluminescent ou enzymatique.

Les méthodes de diagnostic *IN VITRO* dans lesquelles ces sondes nucléotidiques sont mises en oeuvre pour la détection de synthèses aberrantes ou d'anomalies génétiques, telles que la perte d'hétérozygotie et le réarrangement génétique, au niveau des séquences nucléiques codant pour un polypeptide récepteur de l'IL-13 ou un fragment biologiquement actif, sont incluses dans la présente invention. Un tel type de méthode comprend :
- la mise en contact d'une sonde nucléotidique de l'invention avec un échantillon biologique dans des conditions permettant la formation d'un complexe d'hybridation entre ladite sonde et la susdite séquence nucléotidique, éventuellement après une étape préalable d'amplification de la susdite séquence nucléotidique ;
- la détection du complexe d'hybridation éventuellement formé ;
- éventuellement le séquençage de la séquence nucléotidique formant le complexe d'hybridation avec la sonde de l'invention.

Les sondes d'ADNc de l'invention sont en outre avantageusement utilisables pour la détection d'anomalies chromosomiques.

Les séquences nucléotidiques de l'invention sont également utiles pour la fabrication et l'utilisation d'amorces oligonucléotidiques sens et/ou antisens pour des réactions de séquençage ou d'amplification spécifique selon la technique dite de PCR (réaction de polymérisation en chaîne) ou toute autre variante de celle-ci.

Les séquences nucléotidiques selon l'invention ont par ailleurs des utilisations dans le domaine thérapeutique, pour la réalisation de séquences antisens, capables de s'hybrider spécifiquement avec une séquence d'acides nucléiques , y compris un ARN messager, utilisables en thérapie génique. L'invention a ainsi pour objet des séquences antisens capables d'inhiber, au moins partiellement, la production de polypeptides récepteurs de l'IL-13, tels que définis précédemment. De telles séquences sont avantageusement constituées par celles qui constituent le cadre de lecture codant pour l'IL-13Rβ ou l'IL-13Rα au niveau du transcript.

Elles sont plus particulièrement utilisables dans le traitement des allergies et de l'inflammation.

Les séquences nucléotidiques selon l'invention peuvent par ailleurs être utilisées pour la production de polypeptides recombinants ayant une activité de récepteur de l'IL-13, tels que précédemment définis.

Ces polypeptides peuvent être produits à partir des séquences nucléotidiques définies ci-dessus, selon des techniques de production de produits recombinants connues de l'homme du métier. Dans ce cas, la séquence nucléotidique utilisée est placée sous le contrôle de signaux permettant son expression dans un hôte cellulaire. L'hôte cellulaire utilisé peut être choisi parmi des systèmes procaryotes, comme les bactéries, ou eucaryotes, comme par exemple les levures, cellules d'insectes, CHO (cellules d'ovaires de hamster chinois) ou tout autre système avantageusement disponible dans le commerce. Un hôte cellulaire préféré pour l'expression des polypeptides de l'invention est constitué par la lignée fibroblastique COS-7 ou COS-3.

Les signaux contrôlant l'expression des polypeptides, tels que les promoteurs, les activateurs ou les séquences de terminaison, sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation.

Les vecteurs d'expression contenant au moins l'une des séquences nucléotidiques définies ci-dessus font également partie de la présente invention.

Dans le cas des cellules COS-7 ou COS-3 la transfection peut être réalisée à partir du vecteur pSE-1, comme décrit dans (17).

L'invention vise en outre les cellules hôtes transfectées par ces vecteurs d'expression. Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

Ces cellules sont utilisables dans une méthode de production d'un polypeptide recombinant de séquence SEQ ID n° 2 ou SEQ ID n°4 ou dérivée, méthode elle-même comprise dans la présente invention, et caractérisée en ce que l'on cultive les cellules transfectées dans des conditions permettant l'expression d'un polypeptide recombinant de séquence SEQ ID n° 2 ou SEQ ID n°4, ou dérivée, et que l'on récupère ledit polypeptide recombinant.

Les procédés de purification utilisés sont connus de l'homme du métier. Le polypeptide recombinant peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono ou polyclonaux spécifiques.

Les anticorps mono- ou polyclonaux capables de reconnaître spécifiquement l'IL-13Rβ ou/et l'IL-13Rα selon la définition donnée précédemment font également partie de l'invention. Des anticorps polyclonaux peuvent être obtenus à partir du sérum d'un animal immunisé contre l'IL-13Rβ ou/et l'IL-13Rα selon les modes opératoires usuels.

Les anticorps monoclonaux peuvent être obtenus selon la méthode classique de culture d'hybridomes décrite par Kôhler et Milstein (Nature, 1975, *256*, 495-497).

Des anticorps avantageux sont des anticorps dirigés contre le domaine extracellulaire de l'IL-13Rβ ou/et de l'IL-13Rα.

Les anticorps selon l'invention sont par exemple des anticorps chimériques, des anticorps humanisés, des fragments Fab et F (ab')2. Ils peuvent également se présenter sous forme d'immunoconjugués ou d'anticorps marqués. Par exemple, ils peuvent être associés à une toxine, telle la toxine diphtérique ou à un produit radioactif Ces immunotoxines peuvent dans ce cas constituer des agents thérapeutiques utilisables pour le traitement de certaines pathologies impliquant une surexpression de l'IL-13Rβ ou/et de l'IL-13Rα. Les anticorps de l'invention, en particulier les anticorps monoclonaux peuvent également être utilisés pour l'analyse par immunocytochimie des récepteurs de l'IL-13 sur des coupes de tissus spécifiques, par exemple par immunofluorescence, par marquage à l'or, ou à la peroxydase.

Ils peuvent ainsi être avantageusement mis en oeuvre dans toute situation ou l'expression de l'IL-13Rβ ou/et de l'IL-13Rα doit être observée comme par exemple une surexpression anormale ou le suivi de la régulation de l'expression membranaire.

L'invention concerne donc également un procédé de diagnostic *IN VITRO* de pathologies corrélées à une expression anormale de l'IL-13Rβ ou/et de l'IL-13Rα, à partir de prélèvements biologiques susceptibles de contenir l'IL-13Rβ ou/et de l'IL-13Rα exprimé à un taux anormal, caractérisé en ce que l'on met en contact au moins un anticorps de l'invention avec ledit prélèvement biologique, dans des conditions permettant la formation éventuelle de complexes immunologiques spécifiques entre l'IL-13Rβ ou/et de l'IL-13Rα et le ou lesdits anticorps et en ce que l'on détecte les complexes immunologiques spécifiques éventuellement formés.

L'invention concerne également un kit pour le diagnostic *IN VITRO* d'une expression anormale de l'IL-13Rβ ou/et de l'IL-13Rα dans un prélèvement biologique ou/et pour la mesure du taux d'expression du récepteur de l'IL-13 dans ledit prélèvement comprenant :
- au moins un anticorps spécifique de l'IL-13Rβ ou/et de l'IL-13Rα, éventuellement fixé sur un support,
- des moyens de révélation de la formation de complexes antigènes/anticorps spécifiques entre l'IL-13Rβ ou/et de l'IL-13Rα et ledit anticorps et/ou des moyens de quantification de ces complexes.

Un autre objet de l'invention concerne une méthode pour l'identification et/ou l'isolement de ligands spécifiques de l'IL-13Rβ ou/et de l'IL-13Rα ou d'agents capables de moduler son activité, caractérisée en ce que l'on met en contact un composé ou un mélange contenant différents composés, éventuellement non-identifiés, avec des cellules exprimant à leur surface l'IL-13Rβ ou/et de l'IL-13Rα, dans des conditions permettant l'interaction entre le récepteur de l'IL-13 et ledit composé, dans le cas où celui-ci posséderait une affinité pour le récepteur, et en ce que l'on détecte et/ou isole les composés liés à l'IL-13Rβ ou/et de l'IL-13Rα, ou ceux capables d'en moduler l'activité biologique.

Dans un mode particulier, cette méthode de l'invention est adaptée à l'identification et/ou l'isolement d'agonistes et d'antagonistes de l'IL-13 pour son récepteur l'IL-13Rβ ou/et de l'IL-13Rα.

L'invention comprend également des compositions pharmaceutiques comprenant comme principe actif un polypeptide répondant aux définitions précédentes, préférentiellement sous forme soluble, associé à un véhicule pharmaceutiquement acceptable.

Un tel polypeptide peut en effet agir en compétition avec l'IL-13Rβ ou/et de l'IL-13Rα exprimé à la surface cellulaire, et constituer de ce fait un antagoniste spécifique de la liaison de l'IL-13 à son récepteur, qui peut avantageusement être mis en oeuvre pour la synthèse d'un médicament destiné à moduler les réactions médiées par l'IL-13 dans des situations pathologiques.

L'invention comprend enfin une méthode de traitement thérapeutique d'affections liées à des réactions immunologiques médiées par l'IL-13, comprenant l'administration à un patient de l'IL-13Rβ ou/et de l'IL-13Rα (ou d'un de leurs fragments biologiquement actif), ou d'un composé capable d'en moduler spécifiquement l'activité biologique, en association avec un véhicule pharmaceutiquement acceptable.

D'autres caractéristiques et avantages de l'invention apparaissent dans la suite de la description avec les exemples et les figures dont les légendes sont représentées ci-après.

### LEGENDE DES FIGURES

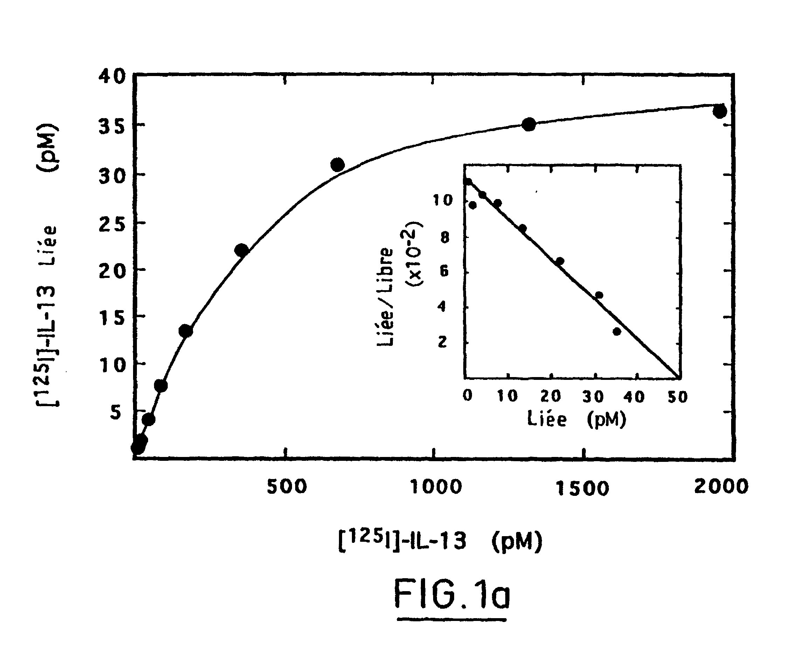

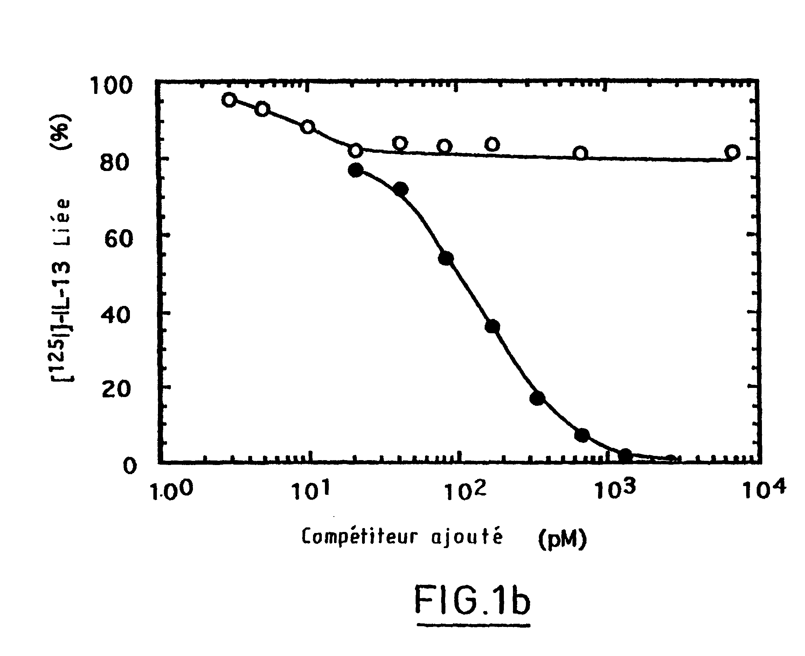

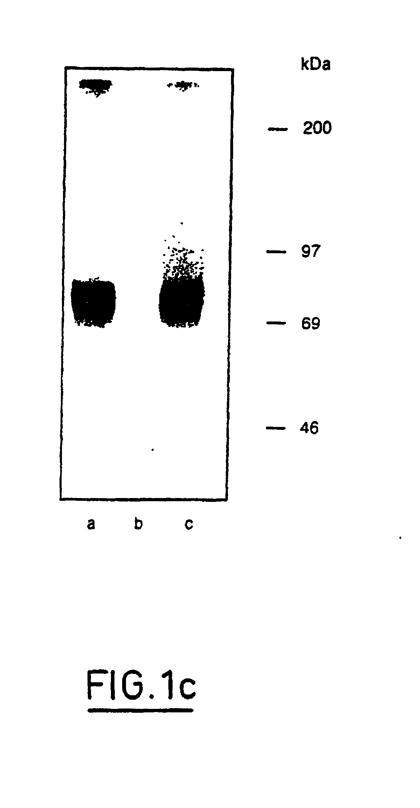

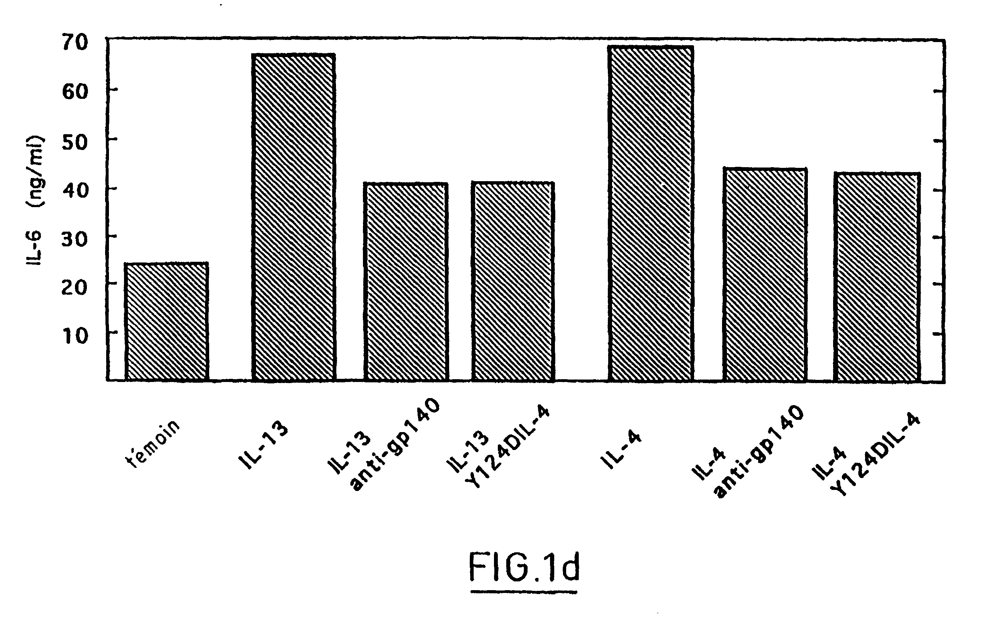

- Figure 1 : caractérisation du récepteur IL-13Rβ humain présent dans les cellules Caki-1.
   a) analyse de Scatchard (cadre inséré) de la courbe de saturation de l'IL-13 marquée à l'[¹²⁵I];
   b) liaison de [¹²⁵I][Phe43]-IL-13-GlyTyrGlyTyr en présence de concentration croissante d'IL-13 non marquée (•) et d'IL-4 (o);
   c) expériences de pontage ("cross-link") en utilisant de l'IL-13 radioactive en l'absence (coulée a) et en présence d'un excédent de 100 fois d'IL-13 non marquée (coulée b) ou d'IL-4 (coulée c);
   d) inhibition de la sécrétion d'IL-6 induite par l'IL-13 et l'IL-4 en présence d'un anticorps monoclonal spécifique de la chaîne IL-4R et de l'antagoniste de IL-4, Y124DIL-4.

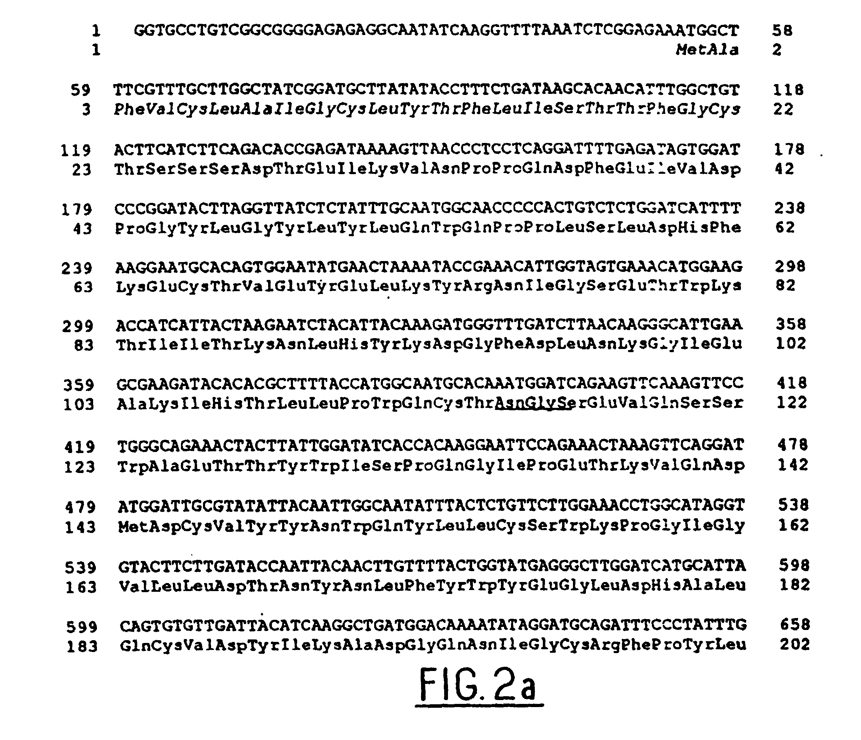

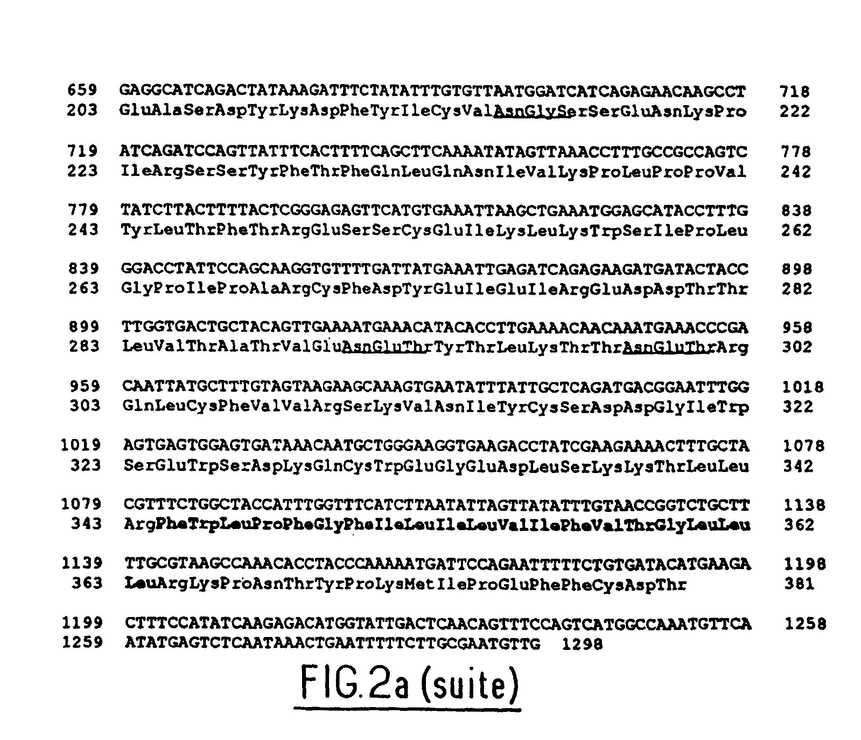

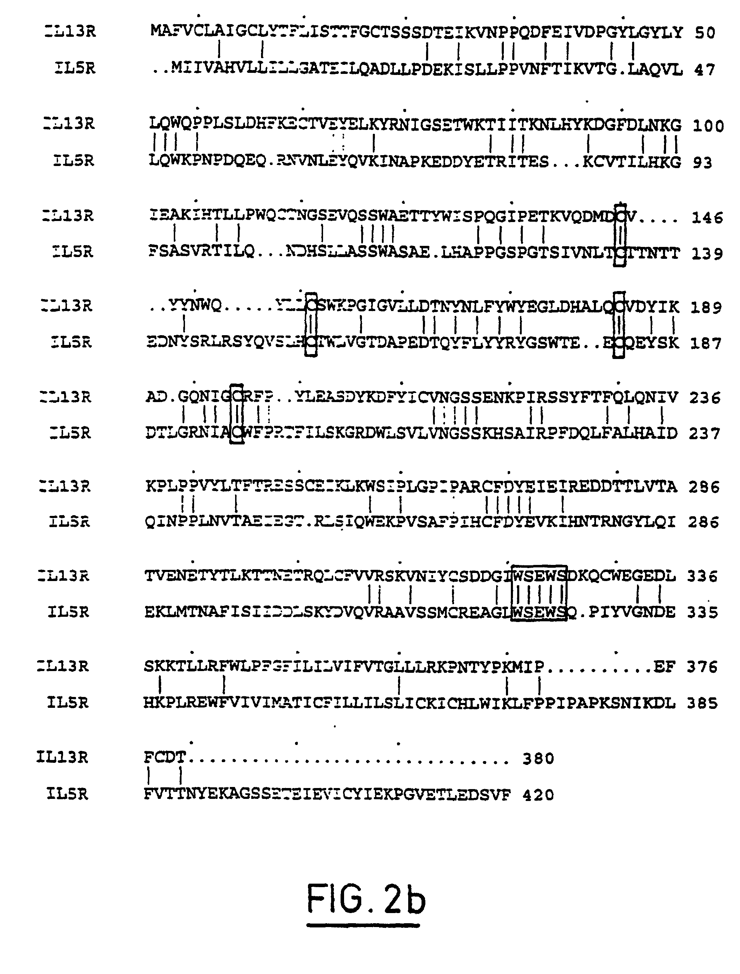

- Figure 2 : Séquence nucléotidique de l'ADNc de l'IL-13Rβ, et comparaison des séquences protéiques de l'IL-5R, l'IL-13Rα mutine et de l'IL-13Rβ.
   a) séquence nucléotidique de l'ADNc de l'IL-13Rβ. Les acides aminés correspondant au peptide signal déduit de la séquence nucléique sont indiqués en italiques et ceux correspondant au domaine transmembranaire sont indiqués en caractères gras. Les sites de N-glycosylation potentiels (Asn-X-Ser/Thr) sont soulignés ;
   b) alignement des acides aminés des séquences de l'IL-13Rβ et de l'IL-5R. Les séquences protéiques de l'IL-13R et IL-5R sont alignées comme décrit précédemment (24). Les résidus cystéine et le motif WSXWS caractéristiques de cette famille de récepteurs sont encadrés.
- Figure 3 : Profils d'expression de l'ARNm de l'IL-13Rβ. L'ARN a été préparé à partir des cellules suivantes : Caki-1 (coulée a), A431 (coulée b), TF-1 (coulée c), U937 (coulée d), Jurkat (coulée e) et IM9 (coulée f).
- Figure 4 : Caractérisation du récepteur IL-13Rβ recombinant de I'IL-13.
   Les cellules COS-7 sont transfectées avec l'ADNc de l'IL-13Rβ et utilisées pour :
   a) des études de liaison de l'IL-13 radiomarquée (cadre inséré), par analyse de Scatchard de la courbe de saturation;
   b) des expériences de pontage en utilisant de l'IL-13 radiomarquée en absence (coulée a) et en présence d'un excès de 100 fois d'IL-13 non marquée (coulée b) ;
   c-d) des expériences de cotransfection en utilisant l'IL-13Rβ cloné, l'IL-4R (gp140) et la chaîne γ suivies par la liaison de l'IL-13 radiomarquée (c) ou de l'IL-4 (d). Les colonnes noires et blanches représentent respectivement la liaison spécifique de l'IL-13 et de l'IL-4.

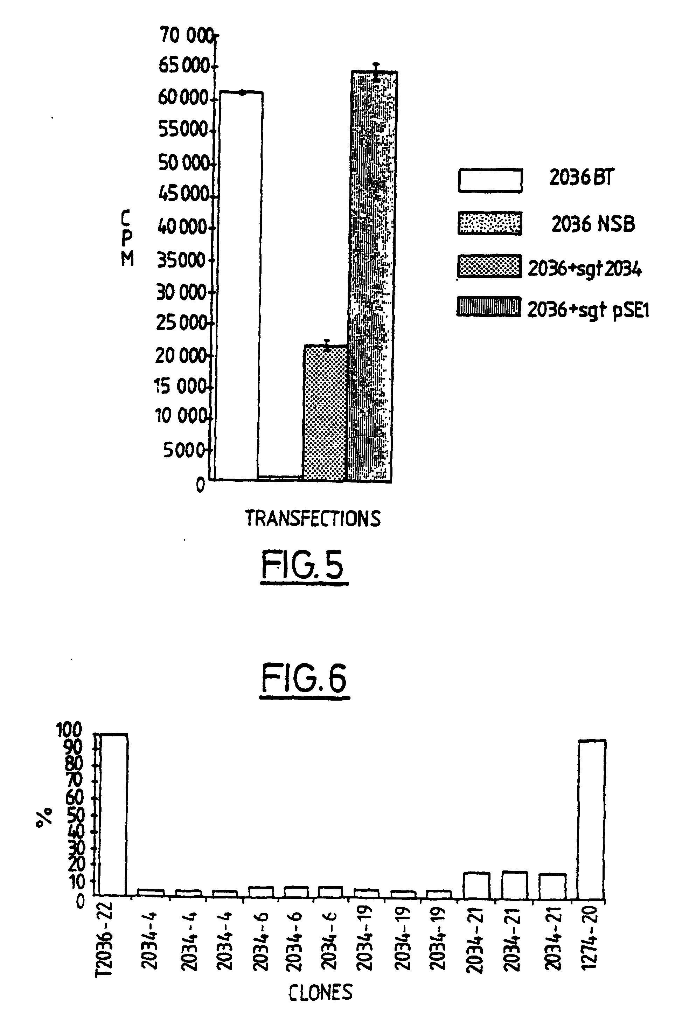

- Figure 5 : Inhibition de la liaison de l'IL-13 sur l'IL-13Rβ par la forme soluble du récepteur (IL-13Rβs) en expression transitoire.
   L'expression d'IL-13Rβs dans le surnageant des cellules transfectées par 2034 est testée par inhibition de la liaison de l'IL-13 sur les cellules transfectées par l'IL-13Rβ (2036). Les surnageants sont testés bruts en les diluant de moitié dans le ligand iodé.
   2036 NSB : liaison non spécifique en présence d'un excès d'IL-13 non marquée 2036 BT : liaison totale sur cellules transfectées avec le 2036
   2036 + sgt 2034 : liaison sur cellules transfectées avec le 2036 en présence de surnageant de cellules transfectées avec le 2034.
   2036 + sgt pSE1 : contrôle
- Figure 6 : Inhibition de la liaison de l'IL-13 sur l'IL-13Rβ par la forme soluble du récepteur (IL-13Rβs) sur des lignées stables.
   T2036-22 : liaison totale sur le clone IL-13Rβ (2036-22) sans présence de surnageant de clone sécrétant l'IL-13Rβs (référence 100 %)
   2034-4
   2034-6
   2034-19 _ 4 clones IL-13Rβs
   2034-21
   1274-20 : en présence de surnageant de cellules CHO n'exprimant pas l'IL-13Rβs (contrôle).

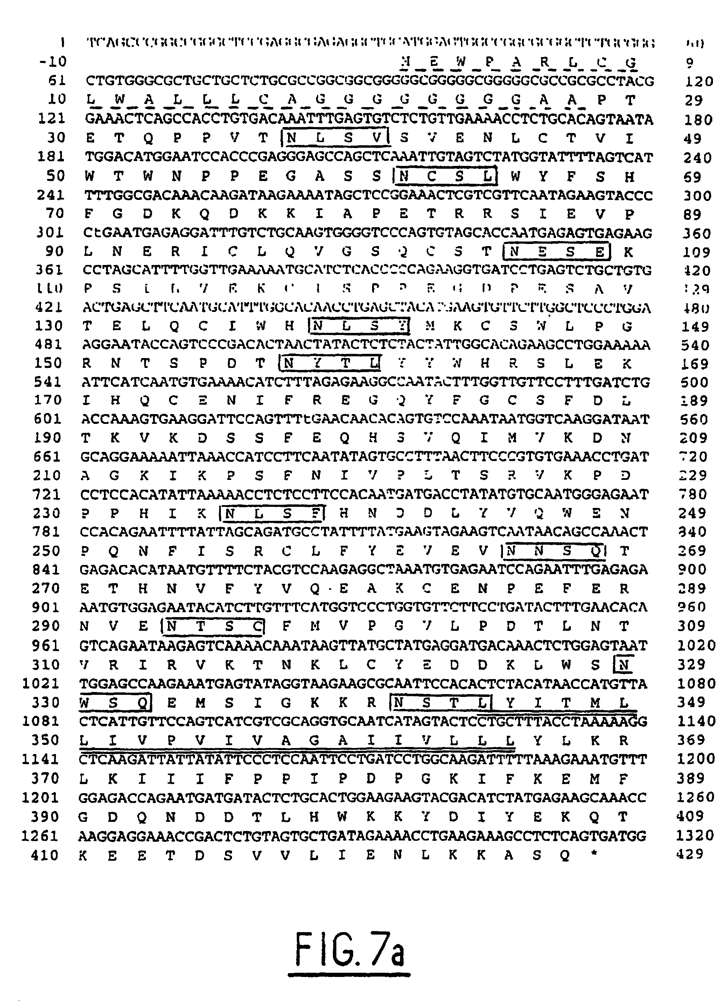

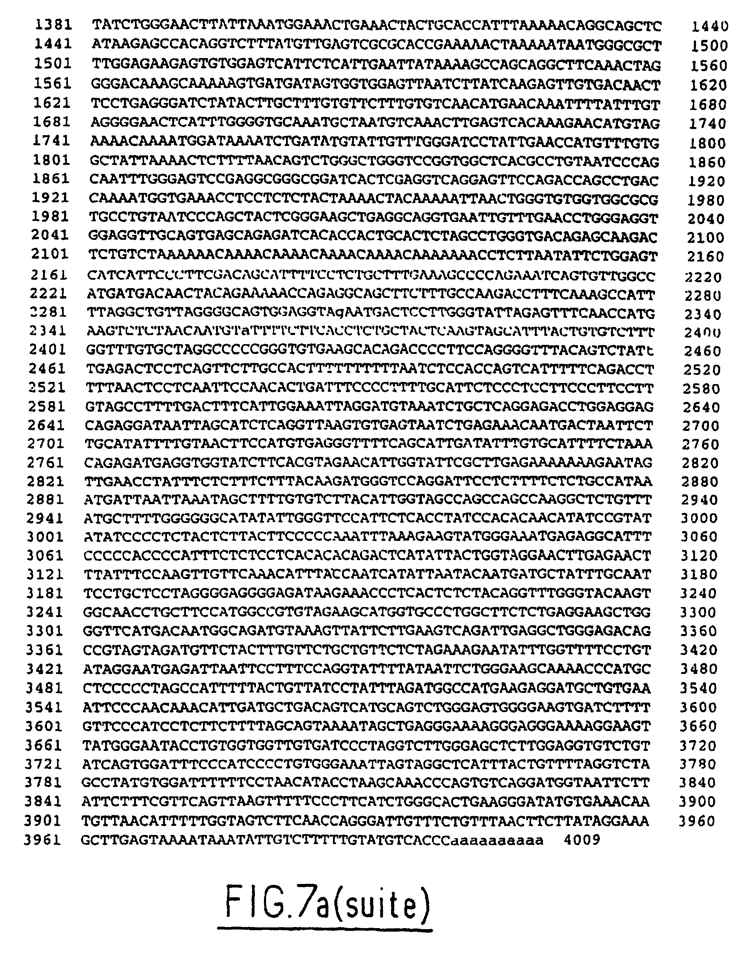

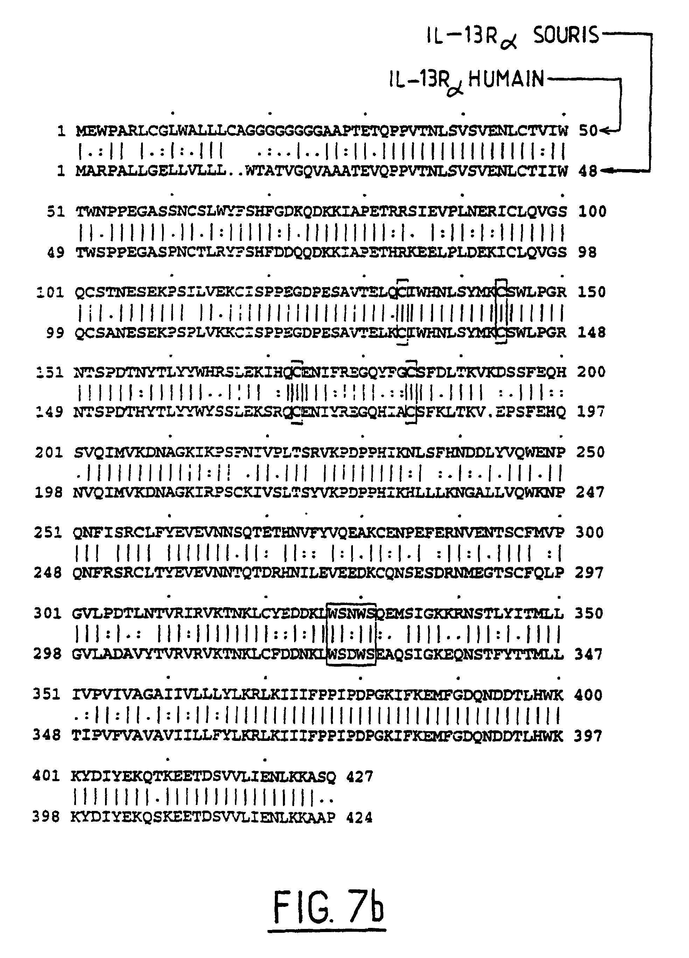

- Figure 7 : Séquence nucléotidique de l'ADNc de l'L-13Rα et comparaison des séquences protéiques de l'IL-13Rαhumain et de l'IL-13Rαmurin
   a) Séquence nucléotique de l'ADNc de l'IL-13Rα. Les acides aminés correspondant au peptide signal déduit de la séquence nucléique sont soulignés par un trait pointillé et ceux correspondant au domaine transmembranaires sont soulignés par un double trait. Les sites de N-glycosylation potentiels (Asn-X-Ser/Thr) sont encadrés.
   b) Alignement des acides aminés de l'IL-13Rαhumain et de l'IL-13Rαmurin. Les séquences protéiques de I'IL-13Rαhumain et de l'IL-13Rα murin sont alignés comme décrit précédemment (24). Les résidus cystéines et le motif WSXWS caractérisdques de cette famille de récepteurs sont encadrés.

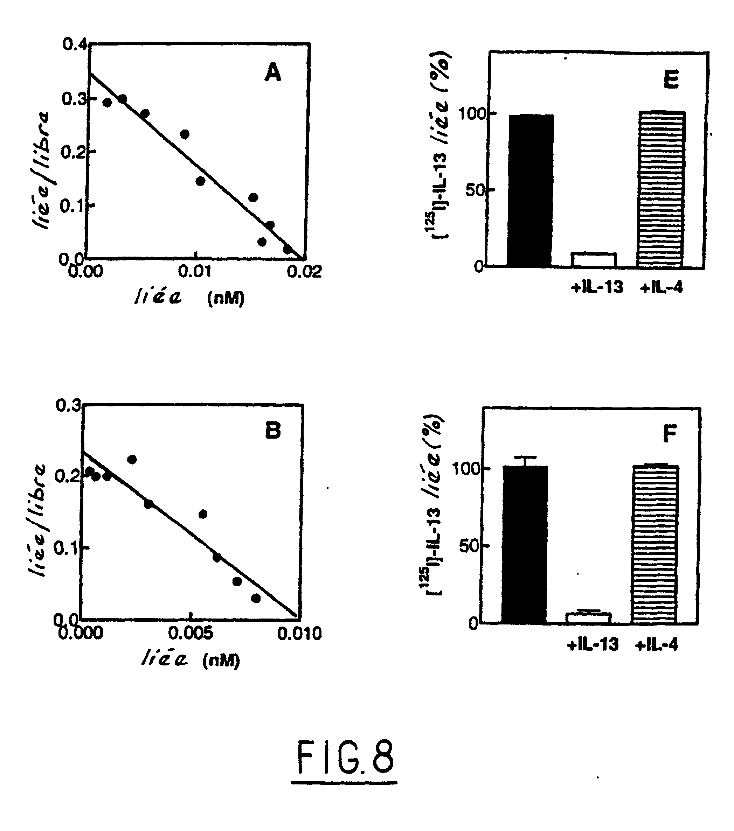

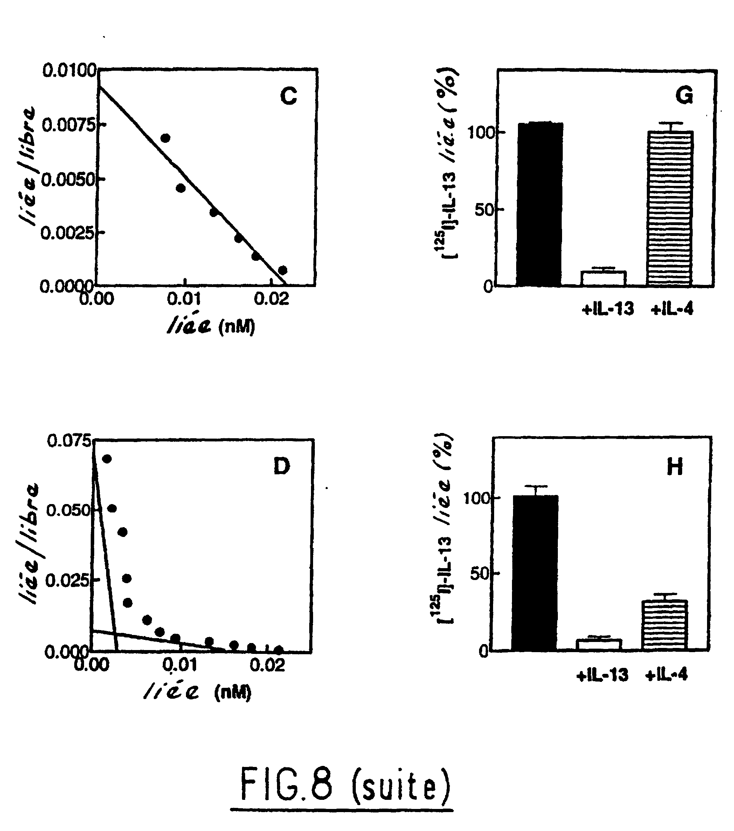

- Figure 8 : Caractérisation du récepteur IL-13Rα recombinant de l'IL-13.
   Les cellules CHO ou COS-3 transfectées avec l'ADNc de l'IL-13Rα ou/et de l'IL-4R et utilisées pour:
   a) des études de liaison de l'IL-13 marquée à l'iode 125, par analyse de Scatchard de la courbe de saturation avec des cellules CHO transfectées avec l'ADNc de l'IL-13Rβ (figure A), transfectées avec l'ADNc de l'IL-13Rβ et l'ADNc de l'IL-4R (figure B), transfectées avec l'ADNc de l'IL-13Rα (figure C) et transfectées avec l'ADNc de l'IL-13Rα et l'ADNc de l'IL-4R (figure D).
   b) des expériences de compétitions de liaisons de l'[¹²⁵I]-IL-13 sur des cellules CHO transfectées avec l'ADNc de l'IL-13Rβ (figure E), transfectées avec l'ADNc de l'IL-13Rβ et l'ADNc de l'IL-4R (figure F), transfectées avec l'ADNc de l'IL-13Rα (figure G) et transfectées avec l'ADNc de l'IL-13Rα et l'ADNc de l'IL-4R (figure H). Les colonnes blanches et hachurées représentent respectivement la liaison spécifique de l'IL-13 radiomarquée en présence d'un excès (1000 fois plus) d'IL-13 ou d'IL-4, les colonnes noires représentent la liaison totale.

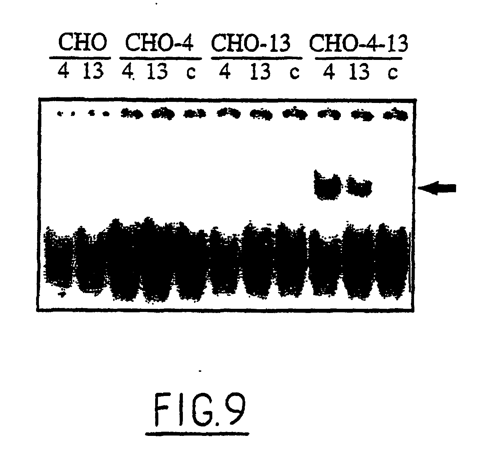

- Figure 9 : comparaison de la mobilité électrophorétique dans l'EMSA d'extraits cellulaires exprimant le récepteur de l'IL-4 seul (CHO-4), le récepteur de l'IL-13Rα seul (CHO-13) ou les récepteurs combinés IL-13Rα etIL-4R (CHO-4-13) après activation des cellules CHO en présence d'IL-4 ou d'IL-13 (4 ou 13), c représentant le témoin non activé..

### MATERIELS ET METHODES

### Expériences de liaison et de pontage :

Les expériences de liaison et de pontage sont réalisées comme décrit pour [¹²⁵I][Phe43]-IL-13-GlyTyrGlyTyr (17).

### Induction de la sécrétion d'IL-6 :

Les cellules Caki-1 (ATCC HTB46) sont mises dans des plaques de 24 puits à une densité de 5.10⁴ cellules/puits et après 3 jours de culture des monocouches confluantes sont lavées trois fois avec du milieu DMEM sans sérum de veau foetal. La stimulation des cellules Caki-1 est réalisée avec 30 ng/ml d'IL-4 ou d'IL-13 en absence ou en présence de Y124DIL-4 ou d'un anticorps monoclonal anti-gp140. La quantité d'IL-6 libérée dans le milieu de culture après 24 heures d'incubation est mesurée par une technique ELISA (Innotest, France).

### Isolement et analyse de l'ADNc de l'IL-13Rβ humain :

L'ARN total a été extrait des cellules Caki-1 comme décrit précédemment (25). L'ARN poly(A) est isolé des ARN totaux avec des billes magnétiques recouvertes d'oligo(dT)₂₅(Dynal). Une banque d'ADNc contenant 2.10⁵ clones a été construite en utilisant la procédure d'amorces adaptateurs (26) et le vecteur pSE-1 (27). La stratégie de clonage pour l'expression qui a été utilisée a été décrite précédemment (17).

### Obtention de l'ADNc de l'IL-13Rβ humain :

Les échantillons d'ARN sont copiés avec la reverse transcriptase et soumis à une PCR (réaction de polymérisation en chaîne) en utilisant une amorce sens correspondant à la séquence + 489 à + 490 et une amorce antisens correspondant à + 52 à + 71 (la numération est faite sur la base de la séquence de l'ADNc montrée sur la figure 2). Les produits amplifiés par PCR sont hybridés avec une sonde complémentaire des séquences + 445 à + 461 de l'ADNc. Les marqueurs de taille sont indiqués à la gauche de la figure.

### Isolement et analyse de l'ADNc de l'IL-13 Rα humain :

### 1) Préparation de la sonde IL-13 Rα murin

### a) Culture des cellules B9 (28)

Les cellules B9 sont cultivées en milieu RPMI ( Gibco ) supplémenté de 10% de sérum de veau foetal et 50 µg / ml de gentamycine.

### b) Préparation de l' ARN des cellules B9.

Les cellules sont lavées deux fois avec du tampon PBS ( phosphate buffer saline, référence 04104040-GIBCO-BRL). Après cemrifugation 10 minutes à 1000 rpm, le culot cellulaire est mis en suspension dans le tampon de lyse de composition suivante : guanidine-thiocyanate 4M ; citrate de sodium 25 mM pH 7 ; sarcosyl 0.5% ; β2-mercaptoéthanol 0.1 M.

La suspension est soniquée à l'aide d'un sonicateur ultra turax no 231256 (JANKE et KUNDEL) à puissance maximale pendant une minute. On ajoute de l'acétate de sodium pH 4 jusqu'à 0.2 M. La solution est extraite avec un volume d'un mélange de phénol / chloroforme ( v / v ; 5 / 1 ).

On précipite à - 20°C l'ARN contenu dans la phase aqueuse à l'aide d'un volume d'isopropanol.Le culot est resuspendu dans le tampon de lyse. La solution est à nouveau extraite avec un mélange phénol / chloroforme et l'ARN est précipité avec de l'isopropanol. Après lavage du culot avec de l'éthanol 70% puis 100%, l'ARN est resuspendu dans de l'eau.

### c) Préparation de l'ADN complémentaire.

L'ADNc est préparé à partir de 5 µg d'ARN total en utilisant une amorce poly T12. On incube l'ARN total dans un volume de 30µl de tampon : Tris HCl 50 mM pH 8.3, MgCl₂ 6 mM, DTT 10 mM, KCl 40 mM, contenant 0.5 mM de chacun des désoxynucléotides triphosphates et 30 unités de Rnasin (Promega), une heure à 37 °C, puis 10 minutes à 50 °C, puis de nouveau 10 minutes à 37 °C, avec 200 unités de l'enzyme transcriptase inverse Rnase H- (Gibco-BRL référence 8064A). La réaction est interrompue en chauffant 10 minutes à 65 °C.

### d) Amplification spécifique d'un fragment de l'ADNc du IL 13Rα de souris par la technique de PCR.

La polymérisation est réalisée avec 6 µl d'ADNc dans 50 µl final avec le tampon de composition suivante : Tris HCl 10mM pH 8.3, MgCl₂ 2.5 mM, KCl 50 mM, 4 dNTP 0.2 mm, 2 µg / ml de chacune des deux amorces nucléiques et de 2.5 u de TAQ ADN polymérase ( Beckman). Les couples d'amorces ont été choisis sur la séquence publiée par Hilton (22).

La réaction est réalisée durant 30 cycles 1 minute à 94°C, 1 minute à 58°C, 4 minutes à 72°C, suivi d'un dernier cycle de 10 minutes à 72°C.

### e) Purification du produit d'amplification PCR.

Après migration sur un gel d'agarose 1% (Sigma) en tampon TAE (Tris HCl 40 mM, EDTA 1mM pH7.9) pendant 1 heure à 100 volts, le gel est coloré en présence de bromure d'ethidium à 1µg / ml dans le même tampon. La bande correspondant au produit d'amplification (fragment d' ADNc de 1027 paires de bases (pB) de l'IL13Rα) est extraite en utilisant un kit Glass Max ( Gibco ).

### f) Préparation de la sonde.

25 ng du fragment de 1027 paires de bases (pB) d'ADNc purifié correspondant au récepteur IL-13Rα de souris sont marqués au phosphore 32 avec le kit BRL " Random Primers DNA labelling systems " à une activité spécifique de 2.4 x 10⁹ dpm / µg ; alternativement, 100 ng sont marqués par Nick-translation en utilisant le kit Boeringher à une activité spécifique de 4 x 10⁸ dpm / µg.

### 2) Isolement et analyse de l'ADNc de l'IL-13Rα humain

### a) Préparation de l'ARN total

L'ARN total a été extrait des cellules Cakil comme décrit précédemment dans le paragraphe 1b.

### b) Purification de l' ARN messager (fraction polyA+).

La purification de la fraction polyA+ de l' ARN est réalisée à l'aide du kit Dynabeads oligo (dT) 25 de DYNAL (référence 610.05) suivant le protocole préconisé par le fabricant. Le principe est basé sur (utilisation de billes polystyrène super-paramagnétique sur lesquelles est attaché un oligonucléotide poly ( dT ) 25. La fraction polyA+ est hybridée sur l'oligonucléotide (dT) 25 couplé aux billes que l'on piège sur un support magnétique.

### c) Northern blot.

5 µg d'ARN messager poly A+ sont déposés sur gel dénaturant 8% formaldéhyde, 1% agarose en tampon MOPS (10mM pH 7.4, EDTA 0.5 mM). Après migration et tranfert sur membrane hybond N+ (Ammersham) en tampon 20 X SSC, l'ARN est fixé en chauffant au four à 80°C sous vide. La membrane est alors préhybridée 2 heures à 42°C dans le tampon suivant : 1 M NaCl ; 30% formamide; 1% SDS ; 5 X Denhart's ; 100 µg / ml d' ADN de sperme de saumon. Après 2 heures de préhybridation, la membrane est hybridée dans le même tampon avec une concentration de sonde IL-13Rα de souris préparée par "random priming" de 2.5 10⁶ dpm / ml, pendant 16 heures. La membrane est ensuite lavée 2 fois pendant 30 minutes dans le tampon 2 x SSC 0.1% SDS à température ambiante puis pendant 2 heures à 50°C dans le même tampon. Après 4 jours d' exposition dans une cassette (Molecular Dynamics), le northern blot est analysé à " l'Instant Imager " (Molecular Dynamics). Un transcrit majoritaire de 4200 paires de bases (pB) et un doublet de 1500 pB et 2000 pB sont détectés dans les cellules Cakil, U373 et U937.

### Caractérisation des propriétés de l'IL-13Rβ et de l'IL-13Rα humain :

Les cellules COS-7 ou CHO sont transfectées dans des boîtes de Pétri comme décrit précédemment (17). 24 heures plus tard, les cellules sont trypsinisées et mises en culture dans des plaques de 24 puits à une densité de 8.10⁴ cellules/puits. Après une culture de 48 heures à 37°C, les cellules sont utilisées pour des expériences de liaison (des essais réalisés en triplicata montrent une variation de moins de 10 %) avec de l'IL-13 iodée comme décrit (17). Pour la transfection, les cellules COS-7 ou CHO ont été transfectées dans des plaques de 25 cm² en utilisant 0,6 mg de différents plasmides. Après 24 heures, les monocouches cellulaires sont trypsinisées et mises en culture dans des plaques à 12 puits à raison de 8.10⁴ cellules/puits. Trois jours plus tard, les expériences de liaison et de compétition sont réalisées avec de l'IL-13 marquée et avec de l'IL-13 et/ou de l'IL-4 non marquées. Les résultats sont représentatifs d'au moins trois expériences réalisées indépendamment.

Comparaison des mobilités électrophorétiques dans l'"EMSA" des extraits nucléiques de cellules exprimant l'IL-13Rα et/ou l'IL-4R humains : 2.10⁶ cellules CHO sont réparties dans des boîtes de Pétri de diamètre 10 cm. 24 heures plus tard, les cellules sont transfectées avec 6 µg d'ADN plasmide (34). Après 48 heures, les cellules sont incubées à 37°C pendant 30 minutes dans 3 ml de milieu avec ou sans IL-13 ou IL-4 à une concentration de 100 ng par ml. Les cellules sont ensuite rincées deux fois avec un tampon PB5-0,5 mM d'EDTA puis resuspendues dans 1,2 ml de PB5. Les cellules sont ensuite centrifugées et les extraits cellulaires préparés comme décrit dans (35). Les EMSA sont ensuite réalisées comme décrit dans (36) avec de 10 à 20 µg d'extraits cellulaires et avec une sonde oligonucléotidique radiomarquée au ³²P (50000-100000 cpm), sonde correspondant à l'élément Cε du promoteur Cε humain (37). La sonde oligonucléotidique synthétisée présente la séquence suivante :

### EXEMPLES

### EXEMPLE 1:

### Analyse de l'expression de l' IL-13Rβ humain à la surface des cellules Caki-1

Il a récemment été découvert que des cellules de carcinome rénal humain exprimaient en plus des récepteurs communs à l'IL-4 et à l'IL-13, un large excès de récepteurs spécifiques de l'IL-13 (21).Sur la base de ces résultats, un échantillon de lignées cellulaires de carcinome humain a été étudié pour la fixation de l'IL-13 comme décrit précédemment (17). Une lignée particulière, Caki-1 (ATCC HTB46), qui exprime un nombre particulièrement important de sites de liaisons pour l'IL-13, a été analysée plus en détail. Les courbes de Scatchard obtenues à partir d'expériences de saturation montrent la présence de sites de liaison avec une Kd de 446±50 pM et une capacité de 7,2.10⁴ récepteurs/cellule (figure 1a). Dans des expériences de compétition, l'IL-13 non marquée déplace totalement l'IL-13 marquée d'une façon dose dépendante, alors que l'IL-4 déplace avec une haute affinité environ 10 % de l'IL-13 marquée. Des concentrations plus importantes d'IL-4 (supérieures à 100 nM) ne déplacent pas les 90 % restants d'IL-13 liée (figure 1b).

Ces résultats sont en concordance avec l'existence de deux sites l'un partagé par les deux cytokines, l'autre spécifique de l'IL-13. Les expériences de pontage par affinité de l'IL-13 montrent un complexe d'environ 70 kDa, qui coïncide avec le complexe observé dans des expériences de pontage similaire avec l'IL-13 dans différents types cellulaires (17,21). L'IL-13 marquée est complétement déplacée du complexe par l'IL-13 mais pas par l'IL-4, ce qui est accord avec les expériences de compétition (figure 1c).

### EXEMPLE 2 :

### Analyse de la sécrétion d'IL-6 induite par l'IL-4 ou l'IL-13.

Les auteurs de l'invention ont analysé la sécrétion induite par l'IL-4 ou l'IL-13 sur des cellules Caki-1. Les deux cytokines induisent la sécrétion de niveaux similaires d'IL-6, et la sécrétion est inhibée par des anticorps spécifiques de la chaîne α de l'IL-4R et par l'antagoniste Y124DIL-4 (figure 1d). Ceci suggère que les récepteurs partagés par les deux cytokines dans les cellules Caki-1 sont responsables de l'induction de la sécrétion d'IL-6. Des résultats similaires sont observés quand la phosphorylation du complexe protéique IRS1/4PS (18) induite - par l'IL-4 et l'IL-13 est analysée en présence ou en absence d'anticorps anti-IL-4R et d'antagoniste de l'IL-4.

Ces résultats pris dans leur ensemble suggèrent que le complexe récepteur IL-4/IL-13 exprimé dans les cellules Caki est identique à ce qui avait été précédemment décrit et que la protéine liant l'IL-13 (IL-13Rβ) qui est surexprimée, est un composant du récepteur responsable de la reconnaissance de l'IL-13 dans un complexe fonctionnel qui inclut IL-4R.

Ces cellules ont donc été utilisées comme source d'ARN messager pour le clonage de cette entité liant l'IL-13.

### EXEMPLE 3:

### Clonage de la sous unité primaire du récepteur de l'IL-13 (IL-13Rβ)

La stratégie de clonage et d'expression qui a été utilisée a été précédemment décrite (17). Une banque d'ADNc contenant 2.10⁵ clones recombinants a été construite (26) à partir des cellules Caki-1 . La banque a été divisée en lots de 1000 ADNc, dont l'ADN de chaque lot sous forme de plasmide a été introduit dans des cellules COS-7 (29). La liaison d'IL-13 marquée aux cellules COS-7 transfectées permet d'identifier les lots de clones codant un récepteur de l'IL-13. Les lots positifs ont été répartis et recriblés jusqu'à l'identification d'un clone unique capable de réaliser la synthèse d'une protéine de surface cellulaire apte à lier l'IL-13. Deux ADNc de l'IL-13Rβ indépendants ont été finalement isolés. La séquence nucléotidique complète de l'ADNc de l'IL-13Rβ et la séquence d'acides aminés qui en est déduite sont montrées sur la figure 2a. L'ADNc a une longueur de 1298 bases en excluant la queue poly-A et a une courte région non traduite en 3' de 106 bases. Un signal canonique de polyadenylation AATAAA se trouve à la place prévue. Le cadre de lecture ouvert entre les nucléotides 53 et 1192 définit un polypeptide de 380 acides aminés. La séquence code pour une protéine membranaire avec un peptide signal potentiel, un seul domaine transmembranaire et une courte queue intracytoplasmique.

4 sites de N-glycosilation potentiels sont localisés dans la région extracellulaire. Il est important de noter que deux motifs consensus considérés comme des signatures de la famille des récepteurs de cytokine de type II (30) sont aussi présents, le premier étant dérivé d'une structure en boucle de pont disulfure N-terminal, le second étant le motif de type WSXWS localisé à l'extrémité C-terminale de la région extra cellulaire. La très courte séquence cytoplasmique pourrait expliquer pourquoi c'est seulement le complexe récepteur partagé par l'IL-4 et par l'IL-13 dans les cellules Caki qui transduit un signal dans la cellule.

Des études d'alignement démontrent des homologies avec la chaîne α de l'IL-5R humain (51 % de similarité et 27 % d'identité, figure 2b) et à un degré moindre, avec le récepteur de la prolactine. Il est intéressant de noter que le complexe IL-5R est constitué d'une chaîne α qui lie l'IL-5 mais qui a besoin d'une autre protéine, la chaîne β partagée avec les récepteurs de l'IL-3 et du GM-CSF, pour former un récepteur de haute affinité qui est capable de transduire un signal (31).

### EXEMPLE 4:

### Détection des ARN messagers de l'IL-13Rβ humain dans différentes lignées cellulaires

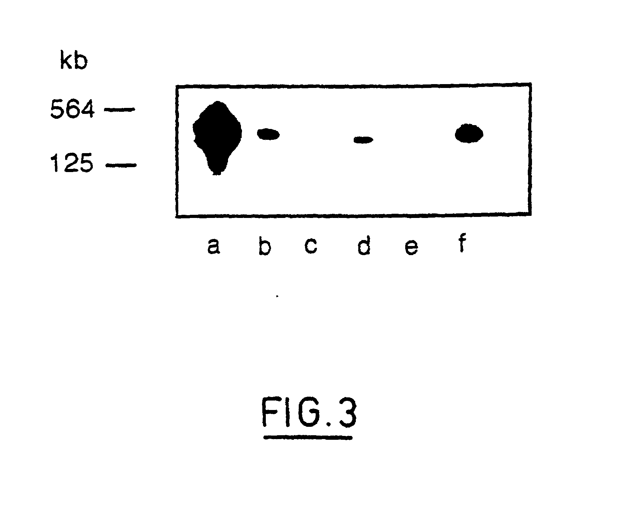

De façon surprenante, dans les cellules Caki-1 des quantités similaires d'ARN messagers de l'IL-13Rβ et IL-4R sont détectées par des analyses de northern blot bien qu'un large excès d'IL-13Rβ soit exprimé. Cette observation suggère qu'il y a une plus forte traduction de cet ARNm par rapport au transcript de l'IL-4R et explique l'absence de détection de l'ARNm de l'IL-13Rβ dans les lignées cellulaires exprimant un faible nombre de sites de liaison de l'IL-13. Des analyses par RT-PCR (figure 3) montrent que le transcript trouvé dans les cellules Caki-1 est aussi présent à des degrés inférieurs dans la lignée kératinocytaire A431, les cellules prémyéloïdes TF-1, les cellules prémocytiques U937, et la lignée cellulaire B IM9. Aucun transcript n'est détecté dans la lignée cellulaire T Jurkat ni dans la lignée cellulaire pré-B NALM6. Ces résultats sont en accord avec des études de liaison de I'IL-13 sur ces mêmes lignées précédemment décrites par les auteurs de la présente invention (17), et avec les cibles biologiques connues de I'IL-13.

### EXEMPLE 5 :

### Analyses de liaison réalisées sur des cellules COS-7 transfectées avec l'ADNc de l'IL-13Rβ humain

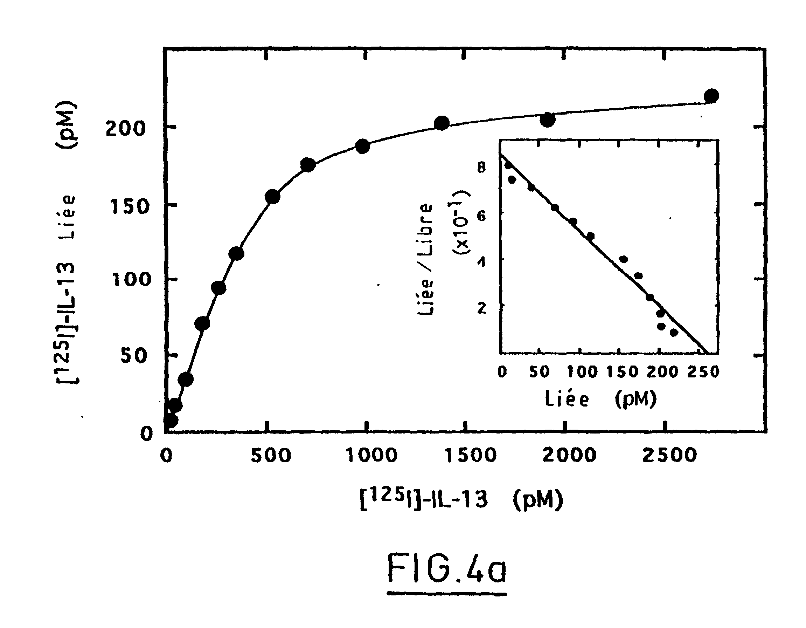

Les cellules COS-7 transfectées avec l'ADNc isolé codant pour l'IL-13Rβ lient spécifiquement I'IL-13 marquée. L'analyse de Scatchard de la courbe de saturation montre un seul site composant avec une Kd d'une valeur de 250±30 pM et une capacité de liaison maximale de 5,6.10⁵ récepteurs/cellule (figure 4a).

L'affinité du récepteur recombinant est en bon accord avec la valeur de Kd de 446 pM pour l'IL-13Rβ dans les cellules Caki-1 et pour ce qui a été décrit dans plusieurs autres cellules (17). Donc, malgré l'homologie de séquence avec la chaîne α de l'IL-5R, le récepteur cloné se comporte différemment puisqu'il n' a pas besoin d'une seconde chaîne pour reconstituer un site de liaison de haute affinité.

Il est intéressant de noter que la protéine liant l'IL-15 récemment décrite possède de la même façon la caractéristique de lier avec une forte affinité l'IL-15, en l'absence des deux autres composants du complexe IL-15R (32).

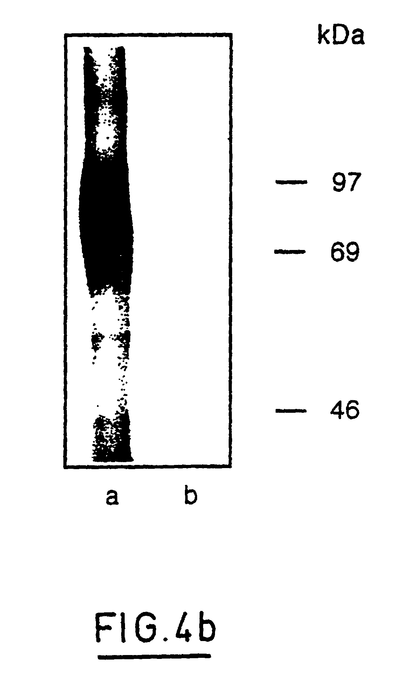

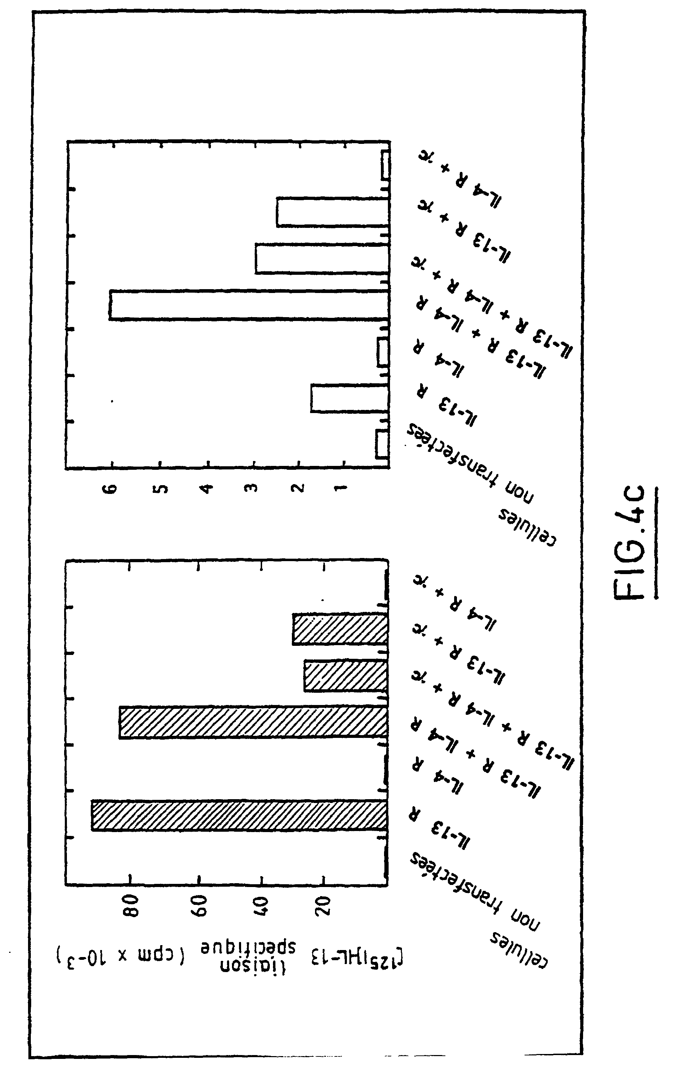

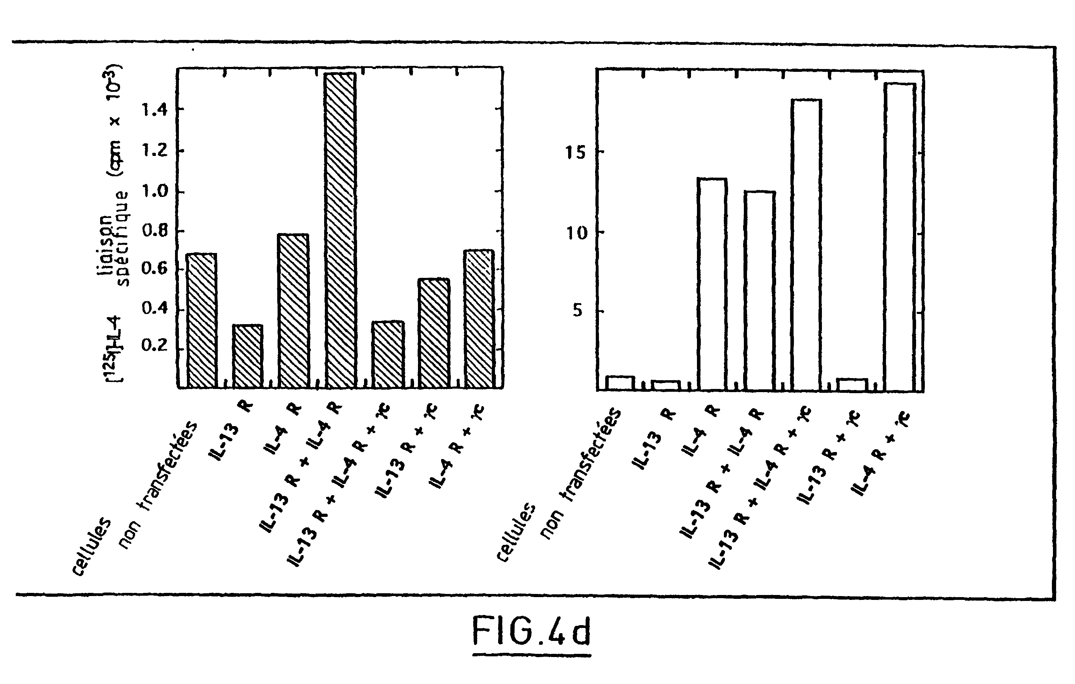

Dans des expériences de compétition, l'IL-13 est capable d'inhiber la liaison de l'IL-13 marquée au récepteur cloné, avec une constante d'inhibition (Ki) de 1,5 ± 0,5 nM, alors que l'IL-4 n'inhibe pas la liaison. La pharmacologie du récepteur cloné est donc similaire à celle de l'IL-13Rβ présent dans les cellules Caki-1. Des expériences de pontage montrent une bande radiomarquée de 70 kDa. Cette bande a la même mobilité que celle observée dans les cellules Caki ainsi que dans d'autres cellules (17). Ce complexe correspond très probablement à la bande de 60-70 kDa observée en plus de la bande de 140 kDa de l'IL-4R dans les expériences de pontage réalisées avec l'IL-4 marquée. Ceci pourrait aussi suggérer qu'il existe une forte interaction entre les deux protéines dans le complexe récepteur fonctionnel. Les auteurs de la présente invention ont donc vérifié si l'IL-13Rβ et l'IL-4R interagissent dans la membrane cellulaire pour reconstituer un récepteur qui permet une compétition croisée des deux cytokines. Les résultats d'expérience de coexpression sont montrés dans la figure 4,c et d.

Il apparait clairement que l'expression des deux récepteurs soit isolée soit simultanée, résulte en un grand nombre de récepteurs qui reconnaissent spécifiquement l'une ou l'autre des deux cytokines. Toutefois, lorsqu'ils sont exprimés ensemble, un petit nombre de récepteurs (5 à 10 %) est capable de reconnaître les deux cytokines. La cotransfection de la chaîne γc avec l'IL-4R et l'IL-13Rβ ne procure pas une augmentation dans le nombre de sites de liaison partagés. Ces résultats suggèrent que les chaînes de l'IL-13Rβ et IL-4R peuvent interagir l'une avec l'autre dans la membrane cellulaire pour reconstituer un récepteur pour lequel l'IL-13 et l'IL-4 peuvent être en compétition. Le faible pourcentage de récepteurs reconstitués est un argument en faveur de la présence d'une autre protéine (IL-13Rα) en quantités limitantes dans les cellules COS, nécessaire pour la reconstitution du complexe récepteur auquel se lient en compétitivité l'IL-13 et l'IL-4.

Les résultats obtenus dans les expériences de transfection avec la chaîne γc démontrent que cette protéine n'est pas le facteur limitant qui avait été suggéré auparavant (15). Cette conclusion est aussi supportée par l'absence d'ARN messager de γc dans les cellules Caki-1 (21).

Une autre raison possible pour expliquer le faible nombre de récepteurs reconstitués est l'existence d'une stoechiométrie incorrecte des deux protéines dans la membrane cellulaire. Toutefois, des cotransfections utilisant des quantités relatives différentes d'IL-4R et d'IL-13Rβ ne montrent pas de différence majeure dans le nombre de récepteurs reconstitués. La possibilité qu'il existe un autre IL-13R avec une plus forte capacité à interagir avec IL-4R, a été confirmée chez la souris (22) et chez l'homme par l'isolement de l'ADNc de l'IL-13Rα (cf. EXEMPLE 7). Il doit être noté que l'expression de γc améliore la liaison de l'IL-4 comme cela a été précédemment décrit (19) mais diminue la liaison de l'IL-13, suggérant une interaction complexe entre les différentes chaînes.

### EXEMPLE 6:

### Etude de l'inhibition de la liaison de l'IL-13 à son récepteur membranaire par un récepteur sous forme soluble.

On décrit les résultats en expression transitoire (figure 5) ou sur les lignées stables (figure 6).

Les deux séquences d'ADNc, codant pour l'IL-13Rβ et pour l'IL-13Rβs sont insérées dans le vecteur p7055 à la place de l'ADNc de l'IL-2 (33). Les plasmides résultants sont nommés 2036 et 2034 respectivement.

### a) Expression transitoire

Les cellules CHO sont ensemencées en plaques 12 puits à 3.10⁵ cellules/puits et transfectées le lendemain par la méthode DEAE-Dextran comme pour les cellules COS, soit avec le plasmide 2036 ou 2034, soit avec le plasmide pSE-1 vide comme témoin.

Les cellules sont cultivées pendant trois jours de façon à permettre une accumulation de l'IL-13Rβs dans le surnageant des cellules transfectées avec le plasmide 2034 et une bonne expression de l'IL-13Rβ dans la membrane des cellules transfectées avec le plasmide 2036.

Le surnageant des cellules transfectées avec l'IL-13Rβs (2034) ou le témoin négatif (pSE-1 vide) est alors prélevé et les cellules transfectées avec l'IL-13Rα sont utilisées pour l'étude de l'inhibition de la liaison de l'IL-13.

La liaison de l'IL-13 à la surface des CHO exprimant l'IL-13Rβ (2036) est mesurée en présence ou non de ces surnageants bruts dilués de moitié avec le radioligand ou en présence d'un excès d'IL-13 non radiomarquée (NSB). La liaison s'effectue sur les cellules entières dans un volume final de 500 ml avec 300 pM de radioligand, en triplicata.

### b) Lignées stables

Deux lignées stables de CHO transformées sont obtenues par transfection avec les séquences codantes de l'IL-13Rβ complet (polypeptide de 380 résidus) ou de l'IL-13Rβ sous forme soluble (IL-13Rβs, polypeptide tronqué, correspondant aux résidus 1 à 337 de l'IL-13Rβ). Ces séquences sont insérées dans le vecteur p 7055.

Les cellules CHO-DHFR⁻ sont transfectées avec les plasmides 2036 (IL-13Rβ) et 2034 (IL-13Rβs) et les clones recombinants sélectionnés comme décrit précédemment (33).

L'un des clones CHO-IL-13Rβ (CHO 2036) obtenus, présentant 2 à 5.10⁵ sites par cellules est ensemencé en plaque 12 puits à une densité de 10⁵ cellules par puits et les cellules sont utilisées deux jours près pour des expériences de liaison en présence ou non d'IL-13Rβs.

Pour cela, les clones CHO-IL-13Rβs (CHO 2034) sont ensemencés en boîtes de 6 cm, en triplicata à 5.10⁵ cellules par boîte. Après 3 jours d'accumulation dans le milieu de culture, le milieu (5 ml par boîte) est prélevé pour les études d'inhibition de liaison de l'IL-13 sur l'IL-13Rβ du clone CHO 2036. De la même façon, le surnageant de cellules CHO n'exprimant pas l'IL-13Rβ soluble est prélevé.

La liaison de l'IL-13 à la surface du clone CHO 2036-22 est mesurée en présence ou non de ces surnageants bruts dilués de moitié avec le radioligand, ou en présence d'un excès d'IL-13 non radiomarquée (NSB). La liaison s'effectue en triplicata, sur les cellules entières, dans un volume de 500 ml avec 300 pM de radioligand.

Les histogrammes des figures 5 et 6 représentent l'inhibition de la liaison de l'IL-13 sur l'IL-13Rβ par l'IL-13Rβs. Une inhibition de la liaison de l'IL-13 à son récepteur peut s'observer sur plusieurs clones.

### EXEMPLE 7

### Clonage du récepteur IL13Rα humain.

### a)Préparation de la banque d' ADNc à partir des ARN messagers polyA+ de cellules Caki1.

A partir de 0.5 µg d'ARN messager polyA+, on prépare l'ADN complémentaire simple brin marqué au ³²P. dCTP (L'ADN complémentaire obtenu présente une activité spécifique de 3000 dpm/ng) avec l' amorce synthétique de séquence suivante (comprenant un site BamH1): dans un volume de 30 µl de tampon suivant :

Tris Hcl 50mM pH 8.3, Mgcl₂ 6mM, DTT 10 mM, Kcl 40 mM, contenant 0.5 mM de chacun des désoxynucléiques triphosphates , 30 µCi de dCTP α³² P et 30 U de Rnasin (Proméga). Après une heure d'incubation à 37°C, puis 10 minutes à 50°C, puis de nouveau 10 minutes à 37°C, avec 200 unités de l' enzyme transcriptase inverse Rnase H- (Gibco -BRL), on ajoute 4 µl d'EDTA. La matrice ARN est ensuite dégradée en ajoutant 6 µl d' une solution de NaOH 2, l'incubation durant 5 minutes à 65°C.

Afin d' éliminer l'amorce synthétique, on purifie l'ADN complémentaire sur une colonne de 1 ml de séphacryl S400 ( Phannacia ), équilibrée dans du tampon TE. Les deux premières fractions radioactives sont regroupées et précipitées avec 1/10 de volume d'une solution d'acétate d'ammonium 10M et 2.5 volumes d'éthanol, ceci après une extraction au chloroforme. On allonge ensuite l'ADNc en 5' en ajoutant une "queue" homopolymérique de dG avec 20 unités de l' enzyme terminale transférase ( Pharmacia 27073001 ). Puis, on incube dans 20 µl de tampon de composition suivante : Tris HCl 30 mM pH 7.6 ; chlorure de cobalt 1 mM ; acide cacodylique 140 mM ; DTT 0.1 mM ; dGTP 1mM, pendant 15 minutes à 37°C, puis on ajoute 2 µl d' EDTA 0.5 M. On retraite de nouveau à la soude sans chauffer, on repurifie sur colonne S400, on extrait au chloroforme et on précipite à l' éthanol. Le culot est dissous dans 33 µl de tampon TE. L'étape suivante consiste à apparier le vecteur de clonage pT7T3-18 auquel on a préalablement ajouté une queue homopolymérique de dC après coupure par Pstl, l'ADNc et l' adaptateur. On met en présence l'ADNc (33 µl) avec 75 ng de vecteur pT7/T3-18 (5µl), 120 ng de l' adapteur (1µ l) de séquence suivante (comprenant un site Apal), 10 µl d' une solution de Nacl 200 mM, et on incube pendant 5 minutes à 65°C puis on laisse refroidir le mélange réactionnel jusqu'à température ambiante. L' étape suivante consiste à ligaturer le vecteur de clonage et l'ADNc simple brin dans un volume réactionnel de 100 µl avec 32.5 unités de l' enzyme ADN ligase du phage T4 (Pharmacia) pendant une nuit à 15°C dans un tampon de composition : Tris HCl 50 mM pH 7.5 ; MgCl₂ 10 mM, ATP 1mM. Les protéines sont ensuite éliminées par extraction au phénol suivie d'une extraction au chloroforme, puis on ajoute 1/10 de volume d'une solution d' acétate d' ammonium 10 mM et 2.5 volumes d' éthanol. On centrifuge, le culot est repris dans le tampon de composition Tris acétate 33 mM pH 7.9, acétate de potassium 62.5mM, acétate de magnésium 1 mM et DTT 1mM; le deuxième brin d' ADNc est synthétisé dans un volume de 30 µl avec 30 unités de l'enzyme ADN polymérase du phage T4 (Pharmacia) et un mélange de 1 mM des quatre désoxynucléotides triphosphates ainsi que deux unités de la protéine du gène 32 du phage T4 (Pharmacia) pendant une heure à 37°C. On extrait au phénol et on en élimine les traces en déposant sur une colonne P10 (Biogel P10-200-400 mesh - référence 15011050 - Biorad).

La dernière étape consiste à transformer des cellules *E*. *Coli* MC 1061 par électroporation de l'ADN recombinant à l' aide d' un appareil Biorad Gene Pulser utilisé à 2.5 kV dans les conditions prescrites par le fabricant, puis on cultive les bactéries pendant une heure dans du milieu LB de composition
bactotryptone 10 g/l; extrait de levure 5 g /l; Nacl 10 g /l.

On détermine le nombre de clones indépendants obtenus en étalant une dilution au 1/1000 ème de la transformation après une heure d' incubation sur une boîte de milieu LB additionné de 1.5 % d' agar (p/v) et de 100 µg / ml d' ampicilline' appelé par la suite milieu LB gélosé.

Le nombre de clones indépendants obtenus est de 1 million.

### b) Criblage de la banque d' ADNc.

La totalité de la banque est étalée sur du milieu gélose (boîtes de Pétri de diamètre de 150 mm) revêtu de membranes Biodyne A (PAUL référence BNNG 132). Après une nuit à 37°C, les clones sont transférés par contact sur de nouvelles membranes . Ces dernières sont traitées en les déposant sur du papier Wathman 3 MM imbibé des solutions suivantes : NaOH 0.5 N, Nacl 1.5 M pendant 5 minutes puis Tris HCl 0.5 M pH 8, NaCl 1.5 M pendant 5 minutes. Aprés un traïtement à la protéinase K dans le tampon suivant : Tris HCl 10mM pH8, EDTA 10mM, NaCl 50mM, SDS 0.1%, protéinase K 100µg/ml pendant 30 minutes à 37°C, les membranes sont lavées abondamment dans du tampon 2 X SSC (sodium citrate NaCl), puis séchées au four sous vide à 80°C pendant 20 minutes.

### c) Préhybridation et hybridation des membranes.

Les membranes sont alors préhybridées 2 heures à 42°C dans le tampon suivant : 1 M NaCl ; 30% formamide ; 1% SDS ; 5 X Denhart's ; 100 µg/ml d' ADN de sperme de saumon. Aprés les 2 heures de préhybridation, les membranes sont hybridées dans le même tampon avec une concentration de sonde IL-13Rα de souris préparée par " nick-translation" de 2.5 10⁶ dpm/ml, pendant 16 heures ensuite. Les membranes sont lavées 2 fois 30 minutes dans le tampon 2 x SSC 0.1% SDS à température ambiante puis 2 heures à 50°C dans le même tampon. Après une nuit d' exposition à - 80°C en présence d' un film Kodak X-OMAT, plusieurs clones positifs sont révélés.

### d) Séquençage d'un clone IL-13Rα humain et analyse de la séquence.

La séquence est obtenue à l' aide du kit Applied Biosystem (référence 401628). La séquence nucléique complète de l' ADNc de l' IL13Rα et la séquence d' acides aminés qui en est déduite sont montrées sur la figure 7. L' ADNc a une longueur de 3999 bases en excluant la queue de poly-A et possède une longue région 3' non traduite de 2145 bases.

Un signal canonique de polyadénylation se trouve à la place prévue. Le cadre de lecture ouvert entre les nucléotide 34 et 1851 définit un polypeptide de 427 acides aminés. La séquence code pour une protéine membranaire avec un peptide signal potentiel, et un seul domaine transmembranaire et une courte région intracytoplasmique.

10 sites de glycosilation potentiels sont localisés dans la région extracellulaire. Il est important de noter que deux motifs consensus considérés comme des signatures de la famille des récepteurs de cytokines de type II sont aussi présents, le premier étant dérivé d'une structure en boucle de pont disulfure N- terminal, le second étant le motif de type WSXWS localisé à l'extrémité C-terminale de région extracellulaire.

### EXEMPLE 8

### Analyses de liaison réalisées sur des cellules COS-3 ou CHO transfectées avec l'ADNc de l'IL-13Rα humain.

Les cellules CHO transfectées avec l'ADNc isolé codant pour l'IL-13Rα lient spécifiquement l'IL-13 marquée.

L'analyse de Scatchard de la courbe de saturation montre un seul site composant avec une Kd d'une valeur de 4,5±0,4nM et une capacité de liaison maximale de 26000 récepteurs/cellule (fig. 8C et 8G).

Les résultats des expériences de co-expression sont montrés dans les figures 8D et 8H.

L'analyse des résultats de la figure 8C met en évidence que l'IL-13Rα s'exprime bien dans le clone 2036 des cellules CHO. On peut noter que l'IL-4R déplace 60 % de la liaison de l'IL-13 dans les cellules CHO cotransfectées avec l'ADNc de l'IL-4R et de l'IL-13Rα (figure 8H) mais en tenant compte d'un Kd de 7,5 nM pour l'IL-13Rα on aurait 10 fois plus de sites IL-13Rα que de sites IL-4R.

Les cellules CHO-hIL4R (IL-4Rα humain) exprimant l'hIL-4R transfectées avec l'ADNc codant pour l'hIL-13Rα lient spécifiquement l'IL-13 marquée.

L'analyse de Scatchard de la courbe de saturation montre clairement 2 sites composants, l'un de haute affinité avec une Kd d'une valeur de 23±8,9 pM et une capacité de liaison maximale de 28000 sites/cellule et, l'autre de basse affinité, avec une Kd d'une valeur de 4,2±1,4 nM et une capacité de liaison maximale de 150000 sites/cellule (fig. 8D).

Le deuxième site caractérisé a la même affinité que l'hIL-13Rα (IL-13Rα humain) exprimé seul et correspond aux chaînes IL-13Rα non associées car exprimées en plus grande quantité que l'hIL-4R.

Ces récepteurs à haute affinité reconstitués en présence des 2 chaînes hIL-13Rα et hIL-4R sont capables de reconnaître les 2 cytokines (fig. 8D et 8H).

Ceci est encore plus clair sur les cellules COS/pSE1 co-exprimant les 2 chaînes hIL-13Rα et hIL-4R en quantité comparable où I'IL-4 déplace toute la liaison IL-13.

L'affinité de l'IL-13Rα humain recombinant est comparable à celle décrite pour le récepteur IL-13Rα de souris (2-10nM) (ref 22).

Contrairement à la chaîne hIL-13Rβ, précédemment décrite, l'IL-13Rα humain ne constitue pas seul un site de liaison de haute affinité.

L'IL-13Rα et l'IL-4R interagissent donc dans la membrane cellulaire pour reconstituer un récepteur à haute affinité.

### EXEMPLE 9

### Activation de proteines STAT par l'IL-13 et l'IL-4 dans les cellules CHO co-exprimant l'hIL-13Rα et l'hIL-4R.

Dans les cellules PBMC humaines, l'hIL-4 et l'IL-13 activent 2 tyrosine kinases de la famille des janus, Jak1 et Jak2 qui phosphorylent un facteur latent de transcription, STAT6. Ce facteur activé entre dans le noyau et se lie à des éléments spécifiques dans les promoteurs des gènes régulés par l'IL-4.

Nous avons choisi l'élément Cε du promoteur du Cε humain comme sonde dans un test de changement de mobilité électrophorétique (EMSA) pour démontrer l'activation par l'IL-13 d'un facteur de liaison similaire à STAT6.

Les extraits nucléaires des cellules CHO, exprimant l'IL-13R seul, l'IL-4R seul, ou les 2 chaînes ensemble, stimulées avec 100ng/ml d'IL-13 ou d'IL-4 pendant 30mn à 37°c, sont incubés avec l'élément Cε radiomarqué.

Les extraits nucléaires des cellules co-exprimant l'hIL-13Rα et l'hIL-4R seul, forment un complexe ayant la même mobilité dans l'EMSA que les cellules soient induites avec l'IL-4 ou l'IL-13 (cf. figure 9). Par contre, avec les cellules exprimant l'une ou l'autre chaîne seule, aucun complexe n'est détecté.

Dans les cellules CHO exprimant l'hIL-13Rα et l'hIL-4R, l'IL-13 et l'IL-4 initient donc la même cascade de signalisation.

Le clonage de l'IL-13Rβ et de l'IL-13Rα décrit ici permet d'améliorer la connaissance des facteurs intervenant dans les réponses spécifiquement induites par l'IL-13 par rapport aux réponses induites par l'IL-4. Il permet en outre de disposer d'un outil pour l'étude de la régulation de l'expression du récepteur dans des conditions normales et pathologiques où l'IL-13 joue un rôle clé.

Par ailleurs, la disponibilité de l'ADNc permet de faciliter le clonage d'autres protéines nécessaires pour la reconstitution d'un complexe récepteur IL-4/IL-13 et est également utile pour la fabrication ou la modélisation rationnelle de nouveaux médicaments capables d'être des antagonistes spécifiques des activités de l'IL-13.

### REFERENCES :

1. Minty, A. *et al*., Nature, 1993, *362,* 248-250.
2. McKenzie, A.N. *et al*., Proc. Natl. Acad. Sci. U.S.A, 1993, *90*, 3735-3739.
3. Defrance, T. *et al*., J Exp. Med., 1994, *179*, 135-143.
4. Punnonen, J. *et al*., Proc. Natl. Acad.Sci. (USA), 1993, *90*, 3730-3734.
5. Fior, R. *et al*., Eur. Cytokine Network, 1994, *5*, 593-600.
6. Muzio, M. R. F. *et al*., Blood, 1994, *83,* 1738-1743.
7. De Waal Malefyt, R. *et al*., J. Immunol, 1993, *151*, 6370-6381.
8. Doyle, A. *et al*., Eur. J. Immunol., 1994, *24*, 1441-1445.
9. Montaner, L.J. *et al*., J. Exp. Med., 1993, *178*, 743-747.
10. Sozzani, P. *et al*., J. Biol. Chem., 1995, *270*, 5084-5088.
11. Herbert, J.M. *et al*., Febs Lett., 1993, *328,* 268-270.
12. Derocq, J.M. *et al*., Febs Lett. 1994, *343,* 32-36.
13. Zurawski, G. *et al*., Immunol. Today, 1994, *15*, 19-26.
14. Interleukin-13 for Cytokines in Health and Disease. Eds D.G. Remick and J.S. Frie, Marcel Decker, N.Y. 1996.
15. Zurawski S.M. *et al*., Embo Journal, 1993, *12*, 2663-2670.
16. Aversa, *G. et al*., J. Exp. Med., 1993, *178*, 2213-2218.
17. Vita, N. *et al*., Biol. Chem., 1995, *270*, 3512-3517.
18. Lefoit, S. *et al*., Febs Lett., 1995, *366*, 122-126.
19. Kondo, M. *et al*., Science, 1993, *262*, 1874-1883.
20. Russell, S.M. *et al*., Science, 1993, *262,* 1880-1883.
21. Obiri, N. *et al*., J. Biol. Chem., 1995, *270,* 8797-8804.
22. Hilton, D.J. *et al*., Proc. Natl. Acad. Sci. USA, 1996, *93*, 497-501.
23. Canard, R.E. *et al*., immunology Today, 1996, *17*, **3**, 108-110.
24. Devereux, J. *et al*., Nucleic Acids Res., 1984, *12*, 387-395.
25. Chomczynski, P. *et al*., N. Anal. Biochem., 1987, *162*, 156-159.
26. Caput, D. *et al.*, Proc. Natl. Acad. Sci. USA, 1986, *83*, 1670-1674.
27. Minty, a. *et al*., Eur. Cytokine Network, 1993, *4*, 99-110
28. Labit Le Bouteiller, C. *et al*., J. of Immunol. Methods, 1995, *181*, 1, 29-36.
29. Seed, B. *et al*., Proc. Natl. Acad. Sci. USA, 1987, *84*, 3365-3369.
30. Bazan, J.F. *et al*., Proc. Natl. Acad. Sci. USA, 1990, *87*, 6934-6938.
31. Honjo, T. *et al*., Current Opinion in Cell Biology, 1991, *1*, 201-203.
32. Giri, J.G. *et al*., Embo Journal, 1993, *14*, 3654-3663.
33. Miloux, B. *et al*., Gene, 1994, *149*, 341-344.
34. Sampayrac, L. M. *et al*., PNAS USA, 1981, 78, 7575-7578.
35. Jiang, S-W *et al*., Nucleic Acid Res., 1995, 23, 3607-3608.
36. Köhler, I *et al*., FEBS Letters, 1994, 345, 187-192.
37. Seidel., H.M. *et al*., PNAS USA, 1995, 92, 3041-3045.

## Revendications

1. Polypeptide purifié, comprenant une séquence d'acides aminés choisie parmi :
a) la séquence SEQ ID n° 2,
b) toute séquence dérivée de SEQ ID n° 2 et capable de se lier spécifiquement à l'IL-13 et/ou de participer à la transduction du signal spécifiquement produit par l'IL-13 au niveau de la membrane cellulaire, et/ou capable d'interagir avec le récepteur spécifique de l'IL-4 (IL-4R/gp 140) pour former un complexe capable de lier l'IL-4 et l'IL-13.

2. Polypeptide selon la revendication 1, **caractérisé en ce qu'**il comprend la séquence d'acides aminés SEQ ID n° 2.

3. Polypeptide selon la revendication 1, **caractérisé en ce qu'**il s'agit d'une forme variante du polypeptide de séquence SEQ ID n°2 dans laquelle les 8 résidus C-terminaux sont substitués par les 6 résidus suivants : VRCVTL.

4. Polypeptide selon la revendication 1, **caractérisé en ce qu'**il s'agit d'une forme soluble s'étendant jusqu'au résidu 343.

5. Polypeptide selon la revendication 1, **caractérisé en ce qu'**il s'agit d'une forme soluble s'étendant jusqu'au résidu 337.

6. Séquence d'acides nucléiques isolée codant pour un polypeptide selon l'une quelconque des revendications 1 à 5.

7. Séquence d'acides nucléiques isolée selon la revendication 6, **caractérisée en ce qu'**elle est choisie parmi :
a) la séquence SEQ ID n° 1,
b) les séquences d'acides nucléiques capables de s'hybrider à la séquence SEQ ID n° 1 et codant pour un polypeptide ayant une activité de récepteur β de l'IL-13,
c) les séquences d'acides nucléiques dérivées des séquences a) et b) du fait de la dégénérescence du code génétique.

8. Séquence d'acides nucléiques selon la revendication 7, **caractérisée en ce qu'**elle comprend ou est constituée par l'enchaînement de nucléotides s'étendant du nucléotide n° 1 jusqu'au nucléotide 1081, et préférentiellement jusqu'au nucléotide 1063 sur la séquence SEQ ID n° 1.

9. Polypeptide purifié, comprenant une séquence d'acides aminés choisie parmi :
a) la séquence SEQ ID n° 4,
b) toute sequence dérivèe de SEQ ID n°4 et capable de se lier spécifiquement à IL A3 et/ou de participer à la transduction du signal spécifiquement produit par l'IL-13 au niveau de la membrane cellulaire, et/ou capable d'interagir avec le récepteur specifique de l'IL-4 (IL-4R/gp 140) pour former un complexe capable de lier l'IL-4 et l'IL-13.

10. Polypeptide selon la revendication 9, **caractérisé en ce qu'**il comprend la séquence d'acides aminés SEQ ID n° 4.

11. Polypeptide selon la revendication 10, **caractérisé en ce qu'**il s'agit d'une forme soluble s'étendant jusqu'au résidu 343 et préférentiellement jusqu'aux résidus compris entre 336 et 342.

12. Séquence d'acides nucléiques isolée codant pour un polypeptide selon l'une quelconque des revendications 9 à 11.

13. Séquence d'acides nucléiques isolée selon la revendication 12, **caractérisée en ce qu'**elle est choisie parmi :
a) la séquence SEQ ID n° 3,
b) les séquences d'acides nucléiques capables de s'hybrider à la séquence SEQ ID n° 3 et codant pour un polypeptide ayant une activité de récepteur α de l'IL-13,
c) les séquences d'acides nucléiques dérivées des séquences a) et b) du fait de la dégénérescence du code génétique.

14. Séquence d'acides nucléiques selon la revendication 13, **caractérisé en ce qu'**elle comprend ou est constituée par l'enchaînement de nucléotides s'étendant du nucléotide n° 1 jusqu'au nucléotide 1059, et préférentiellement jusqu'aux nucléotides compris entre 1041 et 1056 sur la séquence SEQ ID n° 3.

15. Vecteur de clonage et/ou d'expression contenant une séquence d'acides nucléiques selon l'une quelconque des revendications 6 à 8 et 12 à 14.

16. Vecteur selon la revendication 15, **caractérisé en ce qu'**il s'agit du plasmide PSE-1.

17. Cellule hôte transfectée par un vecteur selon la revendication 15 ou 16.

18. Cellule hôte transfectée selon la revendication 17, **caractérisée en ce qu'**il s'agit d'une cellule de la lignée COS-7, COS-3 ou CHO.

19. Sonde nucléotidique hybridant spécifiquement avec l'une quelconque des séquences selon les revendications 6 à 8, leurs séquences complémentaires ou les ARN messagers correspondants, **caractérisée en ce qu'**elle comprend l'intégralité de la séquence SEQ ID n° 1 ou de son brin complémentaire.

20. Sonde nucléotidique hybridant spécifiquement avec l'une quelconque des séquences selon les revendications 12 à 14, leurs séquences complémentaires ou les ARN messagers correspondants, **caractérisé en ce qu'**elle comprend l'intégralité de la SEQ ID n° 3 ou de son brin complémentaire.

21. Utilisation d'une sonde comportant au moins 10 nucléotides hybridant spécifiquement dans des conditions de forte stringence avec l'une quelconque des séquences selon les revendications 6 à 8, leurs séquences complémentaires ou les ARN messagers correspondants, pour la détection *in vitro* de transcripts spécifiques des polypeptides selon les revendications 1 à 5.

22. Utilisation d'une sonde comportant au moins 10 nucléotides hybridant spécifiquement dans des conditions de forte stringence avec l'une quelconque des séquences selon les revendications 12 à 14, leurs séquences complémentaires ou les ARN messagers correspondants, pour la détection *in vitro* de transcripts spécifiques des polypeptides selon les revendications 9 à 11.

23. Séquence antisens capable d'inhiber, au moins partiellement, la production de polypeptides selon l'une quelconque des revendications 5 et 9 à 11, **caractérisée en ce qu'**elle est choisie parmi les séquences constituant le cadre de lecture codant pour un polypeptide selon l'une quelconque des revendications 1 à 5 et 9 à 11 au niveau du transcript.

24. Utilisation d'une séquence selon l'une quelconque des revendications 6 à 8 et 12 à 14, pour la réalisation de sondes nucléotidiques de diagnostic ou de séquences antisens utilisables en thérapie génique.

25. Utilisation d'une sonde telle que décrite dans l'une quelconque des revendications 19 à 22, comme outil de diagnostic *in vitro* pour la détection, par hybridation, des séquences d'acides nucléiques codant pour un polypeptide selon l'une quelconque des revendications 1 à 5 ou 9 à 11, dans des échantillons biologiques, ou pour la mise en évidence de synthèses aberrantes ou d'anomalies génétiques telles que la perte d'hétérozygotie ou le réarrangement génétique.

26. Utilisation d'une sonde telle que décrite dans l'une quelconque des revendications 19 à 22, pour la détection d'anomalies chromosomiques.

27. Méthode de diagnostic *in vitro* pour la détection de synthèses aberrantes ou d'anomalies génétiques au niveau des séquences d'acides nucléiques codant pour un polypeptide selon l'une quelconque des revendications 1 à 5 ou 9 à 11, **caractérisée en ce qu'**elle comprend :
- la mise en contact d'une sonde nucléotidique telle que décrite dans l'une quelconque des revendications 19 à 22 avec un échantillon biologique dans des conditions permettant la formation d'un complexe d'hybridation entre ladite sonde et la susdite séquence nucléotidique, éventuellement après une étape préalable d'amplification de la susdite séquence nucléotidique ;
- la détection du complexe d'hybridation éventuellement formé ;
- éventuellement le séquençage de la séquence nucléotidique formant le complexe d'hybridation avec la sonde de l'invention.

28. Utilisation d'une séquence d'acides nucléiques selon l'une quelconque des revendications 6 à 8 et 12 à 14, pour la production d'un polypeptide recombinant selon l'une quelconque des revendications 1 à 5 et 9 à 11.

29. Méthode de production d'un polypeptide recombinant récepteur de l'IL-13, **caractérisée en ce que** l'on cultive des cellules transfectées selon la revendication 17 ou 18 dans des conditions permettant l'expression d'un polypeptide recombinant de séquence SEQ ID n° 2 ou de séquence SEQ ID n° 4 dérivée, et que l'on récupère ledit polypeptide recombinant.

30. Anticorps mono ou polyclonaux, anticorps conjugués, ou leur fragments, **caractérisés en ce qu'**ils sont capables de reconnaître spécifiquement un polypeptide selon l'une quelconque des revendications 1 à 5 et 9 à 11.

31. Utilisation des anticorps selon la revendication précédente, pour la purification ou la détection d'un polypeptide selon l'une quelconque des revendications 1 à 5 et 9 à 11 dans un échantillon biologique.

32. Procédé de diagnostic *in vitro* de pathologies corrélées à une expression anormale du récepteur de l'IL-13, à partir de prélèvements biologiques susceptibles de contenir le récepteur de l'IL-13 exprimé à un taux anormal, **caractérisé en ce que** l'on met en contact au moins un anticorps selon la revendication 30 avec ledit prélèvement biologique, dans des conditions permettant la formation éventuelle de complexes immunologiques spécifiques entre le récepteur de l'IL-13 et le ou lesdits anticorps et **en ce que** l'on détecte les complexes immunologiques spécifiques éventuellement formés.

33. Kit pour le diagnostic *in vitro* d'une expression anormale du récepteur de l'IL-13 dans un prélèvement biologique et/ou pour la mesure du taux d'expression du récepteur de l'IL-13 dans ledit prélèvement comprenant :
- au moins un anticorps spécifique du récepteur de l'IL-13 selon la revendication 30, éventuellement fixé sur un support,
- des moyens de révélation de la formation de complexes antigènes/anticorps spécifiques entre le récepteur de l'IL-13 et ledit anticorps et/ou des moyens de quantification de ces complexes.

34. Méthode pour l'identification et/ou l'isolement de polypeptides selon la revendication 1 ou 9, ou d'agents capables de moduler leur activité, **caractérisée en ce que** l'on met en contact un composé ou un mélange contenant différents composés, éventuellement non-identifiés, avec dès cellules exprimant à leur surface un polypeptide selon la revendication 1 ou 9, dans des conditions permettant l'interaction entre le polypeptide et ledit composé dans le cas où celui-ci posséderait une affinité pour le polypeptide, et **en ce que** l'on détecte et/ou isole les composés liés au polypeptide ou ceux capables d'en moduler l'activité biologique.

35. Composition pharmaceutique comprenant, à titre de principe actif, un polypeptide selon l'une quelconque des revendications 1 à 5 ou 9 à 11.

36. Composition pharmaceutique selon la revendication précédente, **caractérisée en ce qu'**elle comprend un polypeptide selon l'une quelconque des revendications 4, 5 ou 10.

37. Utilisation d'un polypeptide selon l'une quelconque des revendications 1 à 5, pour le criblage d'agents capables de moduler l'activité de l'IL-13Rβ.

38. Utilisation d'un polypeptide selon l'une quelconque des revendications 9 à 11, pour le criblage d'agents capables de moduler l'activité de l'IL-13Rα.

39. Utilisation d'un polypeptide selon l'une quelconque des revendications 1 à 5, pour la fabrication de produits capables de moduler l'activité de l'IL-13Rβ.

40. Utilisation d'un polypeptide selon l'une quelconque des revendications 9 à 11, pour la fabrication de produits capables de moduler l'activité de l'IL-13Rα.

41. Utilisation d'un polypeptide selon la revendication 4, 5 ou 10 pour la synthèse d'un médicament déstiné au traitement des conditions sensible à un effet antagoniste de l'IL-13 antagoniste de l'IL-13.

42. Utilisation d'un polypeptide selon l'une quelconque des revendications 1 à 5 ou 9 à 11 pour la préparation d'une composition pharmaceutique utile pour la régularisation des mécanismes immunologiques et inflammatoires produits par l'IL-13.

## Patentansprüche

1. Gereinigtes Polypeptid umfassend eine Aminosäuresequenz ausgewählt aus:
a) der Sequenz SEQ ID Nr. 2,
b) jede von SEQ ID Nr. 2 abgeleitete Sequenz, die dazu in der Lage ist, sich spezifisch an IL-13 zu binden und/oder an der Transduktion des durch IL-13 spezifisch gebildeten Signals im Bereich der Zellmembran teilzunehmen und/oder mit dem spezifischen Rezeptor von IL-4 (IL-4R/gp 140) in Wechselwirkung zu treten zur Bildung eines Komplexes, der dazu in der Lage ist, IL-4 und IL-13 zu binden.

2. Polypeptid nach Anspruch 1, **dadurch gekennzeichnet, daß** es die Aminosäuresequenz SEQ ID Nr. 2 enthält.

3. Polypeptid nach Anspruch 1, **dadurch gekennzeichnet, daß** es sich um eine Variante des Polypeptids der Sequenz SEQ ID Nr. 2 handelt, bei der die 8 C-terminalen Reste durch die folgenden 6 Reste substituiert sind: VRCVTL.

4. Polypeptid nach Anspruch 1, **dadurch gekennzeichnet, daß** es sich um eine lösliche Form handelt, die sich bis zu dem Rest 343 erstreckt.

5. Polypeptid nach Anspruch 1, **dadurch gekennzeichnet, daß** es sich um eine lösliche form handelt, die sich bis zu dem Rest 337 erstreckt.

6. Isolierte Nucleinsäuresequenz, die für ein Polypeptid nach einem der Ansprüche 1 bis 5 codiert.

7. Isolierte Nucleinsäuresequenz nach Anspruch 6, **dadurch gekennzeichnet, daß** sie ausgewählt ist aus:
a) der Sequenz SEQ ID Nr. 1.
b) Nucleinsäuresequenzen. die dazu in der Lage sind, mit der Sequenz SEQ ID Nr. 1 zu hybridisieren und die für ein Polypeptid codieren, das eine β-Rezeptor-Aktivität für IL-13 aufweist,
c) die von den Sequenzen a) und b) als Folge der Degenerierung des genetischen Codes abgeleiteten Nucleinsäuresequenzen.

8. Nucleinsäuresequenz nach Anspruch 7, **dadurch gekennzeichnet, daß** sie umfaßt oder gebildet ist durch die Nucleotidkette, die sich vom Nucleotid Nr. 1 bis zu dem Nucleotid 1081 und vorzugsweise bis zu dem Nucleotid 1063 der Sequenz SEQ ID Nr. 1 erstreckt.

9. Gereinigtes Polypeptid umfassend eine Aminosäuresequenz ausgewählt aus:
a) der Sequenz SEQ ID Nr. 4,
b) jede von SEQ ID Nr. 4 abgeleitete Sequenz, die dazu in der Lage ist, sich spezifisch an IL-13 zu binden und/oder an der Transduktion des von IL-13 spezifisch erzeugten Signals im Bereich der Zellmembran teilzunehmen und/oder mit dem spezifischen Rezeptor für IL-4 (IL-4R/gp 140) in Wechselwirkung zu treten unter Bildung eines Komplexes, der dazu in der Lage ist, IL-4 und IL-13 zu binden.

10. Polypeptid nach Anspruch 9, **dadurch gekennzeichnet, daß** es die Aminosäuresequenz SEQ ID Nr. 4 umfaßt.

11. Polypeptid nach Anspruch 10, **dadurch gekennzeichnet, daß** es sich um eine lösliche Form handelt, die sich bis zu dem Rest 343 und vorzugsweise bis zu den Resten zwischen 336 und 342 erstreckt.

12. Isolierte Nucleinsäuresequenz codierend für ein Polypeptid nach einem der Ansprüche 9 bis 11.

13. Isolierte Nucleinsäuresequenz nach Anspruch 12, **dadurch gekennzeichnet, daß** sie ausgewählt ist aus:
a) der Sequenz SEQ ID Nr. 3,
b) Nucleinsäuresequenzen, die in der Lage sind, mit der Sequenz SEQ ID Nr. 3 zu hybridisieren und für ein Polypeptid mit einer α-Rezeptor-Aktivität für IL-13 zu codieren,
c) von den Sequenzen a) und b) als Folge der Degenerierung des genetischen Codes abgeleitete Nucleinsäuresequenzen.

14. Nucleinsäuresequenz nach Anspruch 13, **dadurch gekennzeichnet, daß** sie umfaßt oder gebildet ist durch eine Nucleotidkette, die sich von dem Nucleotid Nr. 1 bis zu dem Nucleotid 1059 und vorzugsweise bis zu Nucleotiden zwischen 1041 und 1056 de Sequenz SEQ ID Nr. 3 erstrecken.

15. Klonierungs- und/oder Expressionsvektor enthaltend eine Nucleinsäuresequenz nach einem der Ansprüche 6 bis 8 und 12 bis 14.

16. Vektor nach Anspruch 15, **dadurch gekennzeichnet, daß** es sich um das Plasmid PSE-1 handelt.

17. Wirtszelle, die mit einem Vektor nach Anspruch 15 oder 16 transfiziert ist.

18. Transfizierte Wirtszelle nach Anspruch 17, **dadurch gekennzeichnet, daß** es sich um eine Zelle der Linie COS-7, COS-3 oder CHO handelt.

19. Nucleotidsonde, die spezifisch hybridisiert mit irgendeiner der Sequenzen nach den Ansprüchen 6 bis 8, deren komplementären Sequenzen oder entsprechenden Messenger-RNA, **dadurch gekennzeichnet, daß** sie die vollständige Sequenz SEQ ID Nr. 1 oder ihren komplementären Strang umfaßt.

20. Nucleotidsonde, die spezifisch hybridisiert mit irgendeiner der Sequenzen nach den Ansprüchen 12 bis 14, deren komplementären Sequenzen oder entsprechenden Messenger-RNA, **dadurch gekennzeichnet, daß** sie die vollständige SEQ ID Nr. 3 oder ihren komplementären Strang umfaßt.

21. Verwendung einer Sonde, die mindestens 10 Nucleotide aufweist, die spezifisch unter den Bedingungen der starken Stringenz mit irgendeiner der Sequenzen nach den Ansprüchen 6 bis 8, deren komplementären Sequenzen oder entsprechenden Messenger-RNA hybridisiert, zum *in vitro*-Nachweis von spezifischen Transkripten von Polypeptiden nach den Ansprüchen 1 bis 5.

22. Verwendung einer Sonde, die mindestens 10 Nucleotide enthält, die spezifisch unter den Bedingungen der starken Stringenz mit irgendeiner der Sequenzen nach den Ansprüchen 12 bis 14, deren komplementären Sequenzen oder entsprechenden Messenger-RNA hybridisiert zum *in vitro*-Nachweis von spezifischen Transkripten von Polypeptiden nach den Ansprüchen 9 bis 11.

23. Antisens-Sequenz, die dazu in der Lage ist, mindestens teilweise die Bildung von Polypeptiden nach einem der Ansprüche 5 bis 9 und 11 zu inhibieren, **dadurch gekennzeichnet, daß** sie aus Sequenzen ausgewählt ist, welche den Leserahmen bilden, der für ein Polypeptid nach einem der Ansprüche 1 bis 5 und 9 bis 11 im Bereich der Transkription codiert.

24. Verwendung einer Sequenz nach einem der Ansprüche 6 bis 8 und 12 bis 14 für die Erzeugung von diagnostischen Nucleotidsonden oder von Antisens-Sequenzen, die in der Gentherapie eingesetzt werden können.

25. Verwendung einer Sonde nach irgendeinem der Ansprüche 19 bis 22 als diagnostisches Werkzeug *in vitro* für den Nachweis durch Hybridisierung von Nucleinsäuresequenzen. die für ein Polypeptid nach einem der Ansprüche 1 bis 5 oder 9 bis 11 codieren, in biologischen Proben oder für den Nachweis von Fehlsynthesen oder genetischen Anomalien, wie den Verlust der Heterozygotie oder der genetischen Umlagerung.

26. Verwendung einer Sonde, wie sie in irgendeinem der Ansprüche 19 bis 22 beschrieben ist, für den Nachweis von Chromosomen-Anomalien.

27. *in vitro*-Diagnosemethode zum Nachweis von Fehlsynthesen oder genetischen Anomalien im Bereich von Nucleinsäuresequenzen, die für ein Polypeptid nach einem der Ansprüche 1 bis 5 oder 9 bis 11 codieren, **dadurch gekennzeichnet, daß** sie umfaßt:
- Inkontaktbringen einer Nucleotidsonde, wie sie in irgendeinem der Ansprüche 19 bis 22 beschrieben ist, mit einer biologischen Probe unter Bedingungen, welche die Bildung eines Hybridisierungskomplexes zwischen der Sonde und der genannten Nucleotidsequenz ermöglichen, gegebenenfalls nach einer vorausgehenden Stufe der Vermehrung der Nucleotidsequenz;
- Nachweis des gegebenenfalls gebildeten Hybridisierungskomplexes;
- gegebenenfalls Sequenzierung der Nucleotidsequenz, welche den Hybridisierungskomplex bildet mit der erfindungsgemäßen Sonde.

28. Verwendung einer Nucleinsäuresequenz nach irgendeinem der Ansprüche 6 bis 8 und 12 bis 14 für die Herstellung eines rekombinanten Polypeptids nach irgendeinem der Ansprüche 1 bis 5 und 9 bis 11.

29. Verfahren zur Herstellung eines rekombinanten Rezeptor-Polypeptids für IL-13, **dadurch gekennzeichnet, daß** man nach Anspruch 17 oder 18 transfizierte Zellen unter Bedingungen züchtet, welche die Expression eines rekombinanten Polypeptids der Sequenz ID Nr. 2 oder der davon abgeleiteten Sequenz SEQ ID Nr. 4 ermöglicht, und Gewinnen des genannten rekombinanten Polypeptids.

30. Mono- oder polyclonale Antikörper, konjugierte Antikörper oder deren Fragmente, **dadurch gekennzeichnet, daß** sie dazu in der Lage sind, spezifisch ein Polypeptid nach irgendeinem der Ansprüche 1 bis 5 und 9 bis 11 zu erkennen.

31. Verwendung der Antikörper nach dem vorhergehenden Anspruch für die Reinigung oder den Nachweis eines Polypeptids nach einem der Ansprüche 1 bis 5 und 9 bis 11 in einer biologischen Probe.

32. *in vitro*-Verfahren zur Diagnose von pathologischen Zuständen, die mit einer anormalen Expression des IL-13-Rezeptors verknüpft sind, ausgehend von entnommenen biologischen Proben, welche den in einem anormalen Ausmaß exprimierten IL-13-Rezeptor enthalten können, **dadurch gekennzeichnet, daß** man mindestens einen Antikörper nach Anspruch 30 mit der entnommenen biologischen Probe unter Bedingungen in Kontakt bringt, welche die eventuelle Bildung von spezifischen immunologischen Komplexen zwischen dem IL-13-Rezeptor und dem oder den Antikörpern ermöglichen, und man die gegebenenfalls gebildeten spezifischen immunologischen Komplexe nachweist.

33. Kit für die *in vitro*-Diagnose einer anormalen Expression des IL-13-Rezeptors in einer entnommenen biologischen Probe und/oder für die Bestimmung des Ausmaßes der Expression des IL-13-Rezeptors in der entnommenen Probe, umfassend:
- mindestens einen spezifischen Antikörper für den IL-13-Rezeptor nach Anspruch 30, der gegebenenfalls an einen Träger gebunden ist,
- Mittel zum Nachweis der Bildung von spezifischen Antlgen/Antikörper-Komplexen zwischen dem IL-13-Rezeptor und den Antikörpern und/oder Mittel zur quantitativen Bestimmung dieser Komplexe.

34. Verfahren zur Identifizierung und/oder Isolierung der Polypeptide nach Anspruch 1 oder 9 oder von Mitteln zur Modulierung ihrer Aktivität, **dadurch gekennzeichnet, daß** man eine Verbindung oder eine Mischung, die verschiedene, gegebenenfalls nicht identifizierte Verbindungen enthält, mit Zellen, die an ihrer Oberfläche ein Polypeptid nach Anspruch 1 oder 9 exprimieren, unter Bedingungen in Kontakt bringt, welche eine Wechselwirkung zwischen dem Polypeptid und der Verbindung in dem Fall, da diese eine Affinität für das Polypeptid aufweist, ermöglichen und man die an das Polypeptid gebundenen Verbindungen oder welche dazu in der Lage sind, deren biologische Aktivität zu modulieren, nachweist und/oder isoliert.

35. Pharmazeutische Zubereitung umfassend als Wirkstoff ein Polypeptid nach irgendeinem der Ansprüche 1 bis 5 oder 9 bis 11.

36. Pharmazeutische Zubereitung nach dem vorhergehenden Anspruch, **dadurch gekennzeichnet, daß** sie ein Polypeptid nach irgendeinem der Ansprüche 4, 5 oder 10 umfaßt.

37. Verwendung eines Polypeptids nach irgendeinem der Ansprüche 1 bis 5 für die Trennung von Mitteln, welche die Aktivität von IL-13Rβ zu modulieren in der Lage sind.

38. Verwendung eines Polypeptids nach irgendeinem der Ansprüche 9 bis 11 zur Trennung von Mitteln, die zur Modulierung der Aktivität von IL-13Rα in der Lage sind.

39. Verwendung eines Polypeptids nach irgendeinem der Ansprüche 1 bis 5 für die Herstellung von Produkten, die für die Modulierung der Aktivität von IL-13Rβ geeignet sind.

40. Verwendung eines Polypeptids nach irgendeinem der Ansprüche 9 bis 11 für die Herstellung von Produkten, die für die Modulierung der Aktivität von IL-13Rα geeignet sind.

41. Verwendung eines Polypeptids nach Anspruch 4, 5 oder 10 für die Synthese eines Arzneimittels zur Behandlung von Zuständen, die gegenüber einem antagonistischen Effekt von IL-13 empfindlich sind.

42. Verwendung eines Polypeptids nach irgendeinem der Ansprüche 1 bis 5 oder 9 bis 11 für die Herstellung einer pharmazeutischen Zubereitung zur Regulierung der immunologischen und inflammatorischen Mechanismen, die von IL-13 verursacht worden sind.

## Claims

1. Purified polypeptide comprising an amino acid sequence selected from among:
(a) the sequence SEQ ID No. 2,
(b) any sequence derived from SEQ ID No. 2 and capable of binding specifically to IL-13 and/or of participating in the transduction of the signal specifically produced by IL-13 at the cell membrane, and/or capable of interacting with the specific receptor for IL-4 (IL-4R/gp 140) to form a complex capable of binding IL-4 and IL-13.

2. Polypeptide according to claim 1, **characterised in that** it comprises the amino acid sequence SEQ ID No. 2.

3. Polypeptide according to claim 1, **characterised in that** it is a variant form of the polypeptide of sequence SEQ ID No. 2 wherein the 8 C-terminal residues are substituted by the following 6 residues: VRCVTL.

4. Polypeptide according to claim 1, **characterised in that** it is a soluble form extending up to residue 343.

5. Polypeptide according to claim 1, **characterised in that** it is a soluble form extending up to residue 337.

6. Isolated nucleic acid sequence coding for a polypeptide according to any one of claims 1 to 5.

7. Isolated nucleic acid sequence according to claim 6, **characterised in that** it is selected from among
(a) the sequence SEQ ID No. 1,
(b) the nucleic acid sequences capable of hybridising with the sequence SEQ ID No. 1 and coding for a polypeptide having an IL-13 β-receptor activity,
(c) the nucleic acid sequences derived from sequences a) and b) as a result of the breakdown of the genetic code.

8. Nucleic acid sequence according to claim 7, **characterised in that** it comprises or consists of a series of nucleotides extending from nucleotide no. 1 to nucleotide 1081, and preferably to nucleotide 1063 on the sequence SEQ ID No. 1.

9. Purified polypeptide, comprising an amino acid sequence selected from among:
a) the sequence SEQ ID No. 4,
b) any sequence derived from SEQ ID No. 4 and capable of binding specifically to IL-13 and/or of participating in the transduction of the signal specifically produced by IL-13 at the cell membrane, and/or capable of interacting with the receptor specific for IL-4 (IL-4R/gp 140) to form a complex capable of binding IL-4 and IL-13.

10. Polypeptide according to claim 9, **characterised in that** it comprises the amino acid sequence SEQ ID No. 4.

11. Polypeptide according to claim 10, **characterised in that** it is a soluble form extending up to residue 343 and preferably up to the residues between 336 and 342.

12. Isolated nucleic acid sequence coding for a polypeptide according to any one of claims 9 to 11.

13. Isolated nucleic acid sequence according to claim 12, **characterised in that** it is selected from among:
(a) the sequence SEQ ID No. 3,
(b) the nucleic acid sequences capable of hybridising with the sequence SEQ ID No. 3 and coding for a polypeptide having an IL-13 α-receptor activity,
(c) the nucleic acid sequences derived from sequences a) and b) as a result of the breakdown of the genetic code.

14. Nucleic acid sequence according to claim 13, **characterised in that** it comprises or consists of a series of nucleotides extending from nucleotide no. 1 to nucleotide 1059, and preferably to the nucleotides between 1041 and 1056 on the sequence SEQ ID No. 3.

15. Cloning and/or expression vector containing a sequence of nucleic acids according to any one of claims 6 to 8 and 12 to 14.

16. Vector according to claim 15, **characterised in that** it is the plasmid PSE-1.

17. Host cell transfected by a vector according to claim 15 or 16.

18. Transfected host cell according to claim 17, **characterised in that** it is a cell of the line COS-7, COS-3 or CHO.

19. Nucleotide probe hybridising specifically with any one of the sequences according to claims 6 to 8, the complementary sequences thereof or the corresponding messenger RNAs, **characterised in that** it includes the entire sequence SEQ ID No. 1 or the complementary strand thereof.

20. Nucleotide probe hybridising specifically with any one of the sequences according to claims 12 to 14, the complementary sequences thereof or the corresponding messenger RNAs, **characterised in that** it includes the entire sequence SEQ ID No. 3 or the complementary strand thereof.

21. Use of a probe comprising at least 10 nucleotides hybridising specifically under highly stringent conditions with any one of the sequences according to claims 6 to 8, the complementary sequences thereof or the corresponding messenger RNAs, for *in vitro* detection of specific transcripts of the polypeptides according to claims 1 to 5.

22. Use of a probe comprising at least 10 nucleotides hybridising specifically under highly stringent conditions with any one of the sequences according to claims 12 to 14, the complementary sequences thereof or the corresponding messenger RNAs, for *in vitro* detection of specific transcripts of the polypeptides according to claims 9 to 11.

23. Antisense sequence capable of at least partially inhibiting the production of polypeptides according to any one of claims 5 and 9 to 11, **characterised in that** it is selected from among the sequences that constitute the reading frame coding for a polypeptide according to any one of claims 1 to 5 and 9 to 11 at the transcription level.

24. Use of a sequence according to any one of claims 6 to 8 and 12 to 14, for producing diagnostic nucleotide probes or antisense sequences which can be used in gene therapy.

25. Use of a probe as described in any one of claims 19 to 22, as an *in vitro* diagnostic tool for detecting, by hybridisation, nucleic acid sequences coding for a polypeptide according to any one of claims 1 to 5 or 9 to 11, in biological samples, or for detecting aberrant syntheses or genetic abnormalities such as loss of heterozygosity or genetic rearrangement.

26. Use of a probe as described in any one of claims 19 to 22, for detecting chromosomal abnormalities.

27. Method of *in vitro* diagnosis for detecting aberrant syntheses or genetic abnormalities in the nucleic acid sequences coding for a polypeptide according to any one of claims 1 to 5 or 9 to 11, **characterised in that** it comprises:
- bringing a nucleotide probe as described in any one of claims 19 to 22 into contact with a biological sample under conditions which allow the formation of a hybridisation complex between said probe and said nucleotide sequence, optionally after a prior step of amplifying said nucleotide sequence;
- detecting the hybridisation complex which may be formed;
- optionally sequencing the nucleotide sequence forming the hybridisation complex with the probe according to the invention.

28. Use of a nucleic acid sequence according to any one of claims 6 to 8 and 12 to 14, for producing a recombinant polypeptide according to any one of claims 1 to 5 and 9 to 11.

29. Method of producing a recombinant IL-13 receptor polypeptide, **characterised in that** transfected cells according to claim 17 or 18 are cultivated under conditions which allow the expression of a recombinant polypeptide of sequence SEQ ID No. 2 or the derived sequence SEQ ID No. 4, and said recombinant polypeptide is recovered.

30. Monoclonal or polyclonal antibodies, conjugated antibodies or fragments thereof, **characterised in that** they are capable of specifically recognising a polypeptide according to any one of claims 1 to 5 and 9 to 11.

31. Use of the antibodies according to the preceding claim, for purifying or detecting a polypeptide according to any one of claims 1 to 5 and 9 to 11 in a biological sample.

32. Process for *in vitro* diagnosis of pathological conditions related to abnormal expression of the IL-13 receptor, from biological samples likely to contain the IL-13 receptor expressed at an abnormal level, **characterised in that** at least one antibody according to claim 30 is brought into contact with said biological sample, under conditions which allow the eventual formation of specific immunological complexes between the IL-13 receptor and the antibody or antibodies and **in that** the specific immunological complexes which may be formed are detected.

33. Kit for *in vitro* diagnosis of abnormal expression of the IL-13 receptor in a biological sample and/or for measuring the level of expression of the IL-13 receptor in said sample, comprising:
- at least one antibody specific for the IL-13 receptor according to claim 30, optionally fixed to a carrier,
- means for revealing the formation of specific antigen/antibody complexes between the IL-13 receptor and said antibody and/or means for quantifying these complexes.

34. Method of identifying and/or isolating polypeptides according to claim 1 or 9, or agents capable of modifying their activity, **characterised in that** a compound or a mixture containing different compounds, possibly unidentified, is brought into contact with cells expressing on their surface a polypeptide according to claim 1 or 9, under conditions which allow an interaction between the polypeptide and said compound if the latter has an affinity for the polypeptide, and **in that** the compounds bound to the polypeptide or those which are capable of modifying its biological activity are detected and/or isolated.

35. Pharmaceutical composition comprising, as active principle, a polypeptide according to any one of claims 1 to 5 or 9 to 11.

36. Pharmaceutical composition according to the preceding claim, **characterised in that** it comprises a polypeptide according to any one of claims 4, 5 or 10.

37. Use of a polypeptide according to any one of claims 1 to 5, for screening agents capable of modifying the activity of IL-13Rβ.

38. Use of a polypeptide according to any one of claims 9 to 11, for screening agents capable of modifying the activity of IL-13Rα.

39. Use of a polypeptide according to any one of claims 1 to 5, for preparing products capable of modifying the activity of IL-13Rβ.

40. Use of a polypeptide according to any one of claims 9 to 11, for preparing products capable of modifying the activity of IL-13Rα.

41. Use of a polypeptide according to claim 4, 5 or 10 for synthesising an IL-13 antagonistic drug intended for treating conditions which respond to an IL-13 antagonistic effect.

42. Use of a polypeptide according to any one of claims 1 to 5 or 9 to 11 for preparing a pharmaceutical composition which may be used for regularising immunological and inflammatory mechanisms produced by IL-13.


### LISTE DE SEQUENCES

<110> Sanofi-Synthélabo
<120> Polypeptide récepteur de l'IL-13
<130> BET 96/884
<140> PCT/FR96/01756
   <141> 1996-11-07
<150> FR 95/14424
   <151> 1995-12-06
<160> 4
<170> PatentIn Ver. 2.1
<210> 1
   <211> 1298
   <212> ADN
   <213> Homo sapiens
<400> 1
   ggtgcctgtc ggcggggaga gaggcaatat caaggtttta aatctcggag aaatggcttt 60
   cgtttgcttg gctatcggat gcttatatac ctttctgata agcacaacat ttggctgtac 120
   ttcatcttca gacaccgaga taaaagttaa ccctcctcag gattttgaga tagtggatcc 180
   cggatactta ggttatctct atttgcaatg gcaaccccca ctgtctctgg atcattttaa 240
   ggaatgcaca gtggaatatg aactaaaata ccgaaacatt ggtagtgaaa catggaagac 300
   catcattact aagaatctac attacaaaga tgggtttgat cttaacaagg gcattgaagc 360
   gaagatacac acgcttttac catggcaatg cacaaatgga tcagaagttc aaagttcctg 420
   ggcagaaact acttattgga tatcaccaca aggaattcca gaaactaaag ttcaggatat 480
   ggattgcgta tattacaatt ggcaatattt actctgttct tggaaacctg gcataggtgt 540
   acttcttgat accaattaca acttgtttta ctggtatgag ggcttggatc atgcattaca 600
   gtgtgttgat tacatcaagg ctgatggaca aaatatagga tgcagatttc cctatttgga 660
   ggcatcagac tataaagatt tctatatttg tgttaatgga tcatcagaga acaagcctat 720
   cagatccagt tatttcactt ttcagcttca aaatatagtt aaacctttgc cgccagtcta 780
   tcttactttt actcgggaga gttcatgtga aattaagctg aaatggagca tacctttggg 840
   acctattcca gcaaggtgtt ttgattatga aattgagatc agagaagatg atactacctt 900
   ggtgactgct acagttgaaa atgaaacata caccttgaaa acaacaaatg aaacccgaca 960
   attatgcttt gtagtaagaa gcaaagtgaa tatttattgc tcagatgacg gaatttggag 1020
   tgagtggagt gataaacaat gctgggaagg tgaagaccta tcgaagaaaa ctttgctacg 1080
   tttctggcta ccatttggtt tcatcttaat attagttata tttgtaaccg gtctgctttt 1140
   gcgtaagcca aacacctacc caaaaatgat tccagaattt ttctgtgata catgaagact 1200
   ttccatatca agagacatgg tattgactca acagtttcca gtcatggcca aatgttcaat 1260
   atgagtctca ataaactgaa tttttcttgc gaatgttg                         1298
<210> 2
   <211> 380
   <212> PRT
   <213> Homo sapiens
<400> 2
   Met Ala Phe Val Cys Leu Ala Ile Gly Cys Leu Tyr Thr Phe Leu Ile
   1               5                   10                  15
   Ser Thr Thr Phe Gly Cys Thr Ser Ser Ser Asp Thr Glu Ile Lys Val
               20                  25                  30
   Asn Pro Pro Gln Asp Phe Glu Ile Val Asp Pro Gly Tyr Leu Gly Tyr
   35 40 45
   Leu Tyr Leu Gln Trp Gln Pro Pro Leu Ser Leu Asp His Phe Lys Glu
   50 55 60
   Cys Thr Val Glu Tyr Glu Leu Lys Tyr Arg Asn Ile Gly Ser Glu Thr
   65 70 75 80
   Trp Lys Thr Ile Ile Thr Lys Asn Leu His Tyr Lys Asp Gly Phe Asp
   85 90 95
   Leu Asn Lys Gly Ile Glu Ala Lys Ile His Thr Leu Leu Pro Trp Gln
   100 105 110
   Cys Thr Asn Gly Ser Glu Val Gln Ser Ser Trp Ala Glu Thr Thr Tyr
   115 120 125
   Trp Ile Ser Pro Gln Gly Ile Pro Glu Thr Lys Val Gln Asp Met Asp
   130 135 140
   Cys Val Tyr Tyr Asn Trp Gln Tyr Leu Leu Cys Ser Trp Lys Pro Gly
   145 150 155 160
   Ile Gly Val Leu Leu Asp Thr Asn Tyr Asn Leu Phe Tyr Trp Tyr Glu
   165 170 175
   Gly Leu Asp His Ala Leu Gln Cys Val Asp Tyr Ile Lys Ala Asp Gly
   180 185 190
   Gln Asn Ile Gly Cys Arg Phe Pro Tyr Leu Glu Ala Ser Asp Tyr Lys
   195 200 205
   Asp Phe Tyr Ile Cys Val Asn Gly Ser Ser Glu Asn Lys Pro Ile Arg
   210 215 220
   Ser Ser Tyr Phe Thr Phe Gln Leu Gln Asn Ile Val Lys Pro Leu Pro
   225 230 235 240
   Pro Val Tyr Leu Thr Phe Thr Arg Glu Ser Ser Cys Glu Ile Lys Leu
   245 250 255
   Lys Trp Ser Ile Pro Leu Gly Pro Ile Pro Ala Arg Cys Phe Asp Tyr
   260 265 270
   Glu Ile Glu Ile Arg Glu Asp Asp Thr Thr Leu Val Thr Ala Thr Val
   275 280 285
   Glu Asn Glu Thr Tyr Thr Leu Lys Thr Thr Asn Glu Thr Arg Gln Leu
   290 295 300
   Cys Phe Val Val Arg Ser Lys Val Asn Ile Tyr Cys Ser Asp Asp Gly
   305 310 315 320
   Ile Trp Ser Glu Trp Ser Asp Lys Gln Cys Trp Glu Gly Glu Asp Leu
   325 330 335
   Ser Lys Lys Thr Leu Leu Arg Phe Trp Leu Pro Phe Gly Phe Ile Leu
   340 345 350
   Ile Leu Val Ile Phe Val Thr Gly Leu Leu Leu Arg Lys Pro Asn Thr
   355 360 365
   Tyr Pro Lys Met Ile Pro Glu Phe Phe Cys Asp Thr
   370 375 380
<210> 3
   <211> 4009
   <212> ADN
   <213> Homo sapiens
<400> 3
   tcagcccggc cgggctccga ggcgagaggc tgcatggagt ggccggcgcg gctctgcggg 60
   ctgtgggcgc tgctgctctg cgccggcggc gggggcgggg gcgggggcgc cgcgcctacg 120
   gaaactcagc cacctgtgac aaatttgagt gtctctgttg aaaacctctg cacagtaata 180
   tggacatgga atccacccga gggagccagc tcaaattgta gtctatggta ttttagtcat 240
   tttggcgaca aacaagataa gaaaatagct ccggaaactc gtcgttcaat agaagtaccc 300
   ctgaatgaga ggatttgtct gcaagtgggg tcccagtgta gcaccaatga gagtgagaag 360
   cctagcattt tggttgaaaa atgcatctca cccccagaag gtgatcctga gtctgctgtg 420
   actgagcttc aatgcatttg gcacaacctg agctacatga agtgttcttg gctccctgga 480
   aggaatacca gtcccgacac taactatact ctctactatt ggcacagaag cctggaaaaa 540
   attcatcaat gtgaaaacat ctttagagaa ggccaatact ttggttgttc ctttgatctg 600
   accaaagtga aggattccag ttttgaacaa cacagtgtcc aaataatggt caaggataat 660
   gcaggaaaaa ttaaaccatc cttcaatata gtgcctttaa cttcccgtgt gaaacctgat 720
   cctccacata ttaaaaacct ctccttccac aatgatgacc tatatgtgca atgggagaat 780
   ccacagaatt ttattagcag atgcctattt tatgaagtag aagtcaataa cagccaaact 840
   gagacacata atgttttcta cgtccaagag gctaaatgtg agaatccaga atttgagaga 900
   aatgtggaga atacatcttg tttcatggtc cctggtgttc ttcctgatac tttgaacaca 960
   gtcagaataa gagtcaaaac aaataagtta tgctatgagg atgacaaact ctggagtaat 1020
   tggagccaag aaatgagtat aggtaagaag cgcaattcca cactctacat aaccatgtta 1080
   ctcattgttc cagtcatcgt cgcaggtgca atcatagtac tcctgcttta cctaaaaagg 1140
   ctcaagatta ttatattccc tccaattcct gatcctggca agatttttaa agaaatgttt 1200
   ggagaccaga atgatgatac tctgcactgg aagaagtacg acatctatga gaagcaaacc 1260
   aaggaggaaa ccgactctgt agtgctgata gaaaacctga agaaagcctc tcagtgatgg 1320
   agataattta tttttacctt cactgtgacc ttgagaagat tcttcccatt ctccatttgt 1380
   tatctgggaa cttattaaat ggaaactgaa actactgcac catttaaaaa caggcagctc 1440
   ataagagcca caggtcttta tgttgagtcg cgcaccgaaa aactaaaaat aatgggcgct 1500
   ttggagaaga gtgtggagtc attctcattg aattataaaa gccagcaggc ttcaaactag 1560
   gggacaaagc aaaaagtgat gatagtggtg gagttaatct tatcaagagt tgtgacaact 1620
   tcctgaggga tctatacttg ctttgtgttc tttgtgtcaa catgaacaaa ttttatttgt 1680
   aggggaactc atttggggtg caaatgctaa tgtcaaactt gagtcacaaa gaacatgtag 1740
   aaaacaaaat ggataaaatc tgatatgtat tgtttgggat cctattgaac catgtttgtg 1800
   gctattaaaa ctcttttaac agtctgggct gggtccggtg gctcacgcct gtaatcccag 1860
   caatttggga gtccgaggcg ggcggatcac tcgaggtcag gagttccaga ccagcctgac 1920
   caaaatggtg aaacctcctc tctactaaaa ctacaaaaat taactgggtg tggtggcgcg 1980
   tgcctgtaat cccagctact cgggaagctg aggcaggtga attgtttgaa cctgggaggt 2040
   ggaggttgca gtgagcagag atcacaccac tgcactctag cctgggtgac agagcaagac 2100
   tctgtctaaa aaacaaaaca aaacaaaaca aaacaaaaaa acctcttaat attctggagt 2160
   catcattccc ttcgacagca ttttcctctg ctttgaaagc cccagaaatc agtgttggcc 2220
   atgatgacaa ctacagaaaa accagaggca gcttctttgc caagaccttt caaagccatt 2280
   ttaggctgtt aggggcagtg gaggtagaat gactccttgg gtattagagt ttcaaccatg 2340
   aagtctctaa caatgtattt tcttcacctc tgctactcaa gtagcattta ctgtgtcttt 2400
   ggtttgtgct aggcccccgg gtgtgaagca cagacccctt ccaggggttt acagtctatt 2460
   tgagactcct cagttcttgc cacttttttt tttaatctcc accagtcatt tttcagacct 2520
   tttaactcct caattccaac actgatttcc ccttttgcat tctccctcct tcccttcctt 2580
   gtagcctttt gactttcatt ggaaattagg atgtaaatct gctcaggaga cctggaggag 2640
   cagaggataa ttagcatctc aggttaagtg tgagtaatct gagaaacaat gactaattct 2700
   tgcatatttt gtaacttcca tgtgagggtt ttcagcattg atatttgtgc attttctaaa 2760
   cagagatgag gtggtatctt cacgtagaac attggtattc gcttgagaaa aaaagaatag 2820
   ttgaacctat ttctctttct ttacaagatg ggtccaggat tcctcttttc tctgccataa 2880
   atgattaatt aaatagcttt tgtgtcttac attggtagcc agccagccaa ggctctgttt 2940
   atgcttttgg ggggcatata ttgggttcca ttctcaccta tccacacaac atatccgtat 3000
   atatcccctc tactcttact tcccccaaat ttaaagaagt atgggaaatg agaggcattt 3060
   cccccacccc atttctctcc tcacacacag actcatatta ctggtaggaa cttgagaact 3120
   ttatttccaa gttgttcaaa catttaccaa tcatattaat acaatgatgc tatttgcaat 3180
   tcctgctcct aggggagggg agataagaaa ccctcactct ctacaggttt gggtacaagt 3240
   ggcaacctgc ttccatggcc gtgtagaagc atggtgccct ggcttctctg aggaagctgg 3300
   ggttcatgac aatggcagat gtaaagttat tcttgaagtc agattgaggc tgggagacag 3360
   ccgtagtaga tgttctactt tgttctgctg ttctctagaa agaatatttg gttttcctgt 3420
   ataggaatga gattaattcc tttccaggta ttttataatt ctgggaagca aaacccatgc 3480
   ctccccctag ccatttttac tgttatccta tttagatggc catgaagagg atgctgtgaa 3540
   attcccaaca aacattgatg ctgacagtca tgcagtctgg gagtggggaa gtgatctttt 3600
   gttcccatcc tcttctttta gcagtaaaat agctgaggga aaagggaggg aaaaggaagt 3660
   tatgggaata cctgtggtgg ttgtgatccc taggtcttgg gagctcttgg aggtgtctgt 3720
   atcagtggat ttcccatccc ctgtgggaaa ttagtaggct catttactgt tttaggtcta 3780
   gcctatgtgg attttttcct aacataccta agcaaaccca gtgtcaggat ggtaattctt 3840
   attctttcgt tcagttaagt ttttcccttc atctgggcac tgaagggata tgtgaaacaa 3900
   tgttaacatt tttggtagtc ttcaaccagg gattgtttct gtttaacttc ttataggaaa 3960
   gcttgagtaa aataaatatt gtctttttgt atgtcaccca aaaaaaaaa          4009
<210> 4
   <211> 427
   <212> PRT
   <213> Homo sapiens
<400> 4
   Met Glu Trp Pro Ala Arg Leu Cys Gly Leu Trp Ala Leu Leu Leu Cys
   1 5 10 15
   Ala Gly Gly Gly Gly Gly Gly Gly Gly Ala Ala Pro Thr Glu Thr Gln
   20 25 30
   Pro Pro Val Thr Asn Leu Ser Val Ser Val Glu Asn Leu Cys Thr Val
   35 40 45
   Ile Trp Thr Trp Asn Pro Pro Glu Gly Ala Ser Ser Asn Cys Ser Leu
   50 55 60
   Trp Tyr Phe Ser His Phe Gly Asp Lys Gln Asp Lys Lys Ile Ala Pro
   65 70 75 80
   Glu Thr Arg Arg Ser Ile Glu Val Pro Leu Asn Glu Arg Ile Cys Leu
   85 90 95
   Gln Val Gly Ser Gln Cys Ser Thr Asn Glu Ser Glu Lys Pro Ser Ile
   100 105 110
   Leu Val Glu Lys Cys Ile Ser Pro Pro Glu Gly Asp Pro Glu Ser Ala
   115 120 125
   Val Thr Glu Leu Gln Cys Ile Trp His Asn Leu Ser Tyr Met Lys Cys
   130 135 140
   Ser Trp Leu Pro Gly Arg Asn Thr Ser Pro Asp Thr Asn Tyr Thr Leu
   145 150 155 160
   Tyr Tyr Trp His Arg Ser Leu Glu Lys Ile His Gln Cys Glu Asn Ile
   165 170 175
   Phe Arg Glu Gly Gln Tyr Phe Gly Cys Ser Phe Asp Leu Thr Lys Val
   180 185 190
   Lys Asp Ser Ser Phe Glu Gln His Ser Val Gln Ile Met Val Lys Asp
   195 200 205
   Asn Ala Gly Lys Ile Lys Pro Ser Phe Asn Ile Val Pro Leu Thr Ser
   210 215 220
   Arg Val Lys Pro Asp Pro Pro His Ile Lys Asn Leu Ser Phe His Asn
   225 230 235 240
   Asp Asp Leu Tyr Val Gln Trp Glu Asn Pro Gln Asn Phe Ile Ser Arg
   245 250 255
   Cys Leu Phe Tyr Glu Val Glu Val Asn Asn Ser Gln Thr Glu Thr His
   260 265 270
   Asn Val Phe Tyr Val Gln Glu Ala Lys Cys Glu Asn Pro Glu Phe Glu
   275 280 285
   Arg Asn Val Glu Asn Thr Ser Cys Phe Met Val Pro Gly Val Leu Pro
   290 295 300
   Asp Thr Leu Asn Thr Val Arg Ile Arg Val Lys Thr Asn Lys Leu Cys
   305 310 315 320
   Tyr Glu Asp Asp Lys Leu Trp Ser Asn Trp Ser Gln Glu Met Ser Ile
   325 330 335
   Gly Lys Lys Arg Asn Ser Thr Leu Tyr Ile Thr Met Leu Leu Ile Val
   340 345 350
   Pro Val Ile Val Ala Gly Ala Ile Ile Val Leu Leu Leu Tyr Leu Lys
   355 360 365
   Arg Leu Lys Ile Ile Ile Phe Pro Pro Ile Pro Asp Pro Gly Lys Ile
   370 375 380
   Phe Lys Glu Met Phe Gly Asp Gln Asn Asp Asp Thr Leu His Trp Lys
   385 390 395 400
   Lys Tyr Asp Ile Tyr Glu Lys Gln Thr Lys Glu Glu Thr Asp Ser Val
   405 410 415
   Val Leu Ile Glu Asn Leu Lys Lys Ala Ser Gln
   420 425